# EUROPEAN PATENT APPLICATION

(11) **EP 2 626 920 A1**
(43) Date of publication of application: **14.08.2013**
(21) Application number: 11853560.8
(22) Date of filing: 21.12.2011
(51) Int. Cl.: H01L 51/50, C07D 209/86, C07D 209/88

(54) **LIGHT EMITTING ELEMENT MATERIAL AND LIGHT EMITTING ELEMENT**

(30) Priority: 27.12.2010 JP 2010290139
(71) Applicant: Toray Industries, Inc., Tokyo 103-8666 (JP)
(72) Inventor: MATSUKI, Shinichi, Shiga 520-8558 (JP); TANAKA, Daisaku, Shiga 520-8558 (JP); TOMINAGA, Tsuyoshi, Shiga 520-8558 (JP)
(74) Representative: Hoefer & Partner
(86) International application number: PCT/JP2011/079594
(87) International publication number: WO 2012/090806

(57) **Abstract**

The present invention solves the problem of no technology yet being found that establishes durable life as well as low driving voltage and high luminance efficiency in organic thin-film light emitting devices. An object of the present invention is to provide an organic thin-film light emitting device in which the luminance efficiency and durable life are improved while a low driving voltage is maintained by means of a light emitting device material that contains a compound represented by general formula (1).

## Description

### [TECHNICAL FIELD]

The present invention relates to a light emitting device capable of converting electric energy into light, and a light emitting device material to be used for the same. In particular, the present invention relates to a light emitting device capable of being used for areas such as display devices, flat-panel displays, backlight, lighting, interior design, labels, signboards, electrophotography machines, and light signal generators, and also to a light emitting device material to be used for the same.

### [BACKGROUND ART]

Researches on an organic thin-film light emitting device in which electrons injected from a cathode and holes injected from an anode emit light when they are recombined in an organic fluorescent body held by both electrodes have been actively conducted in recent years. This light emitting device is characteristic for high luminance light emission in the form of a thin type and under a low driving voltage, and multicolor light emission due to selection of a fluorescent material, and has been paid attention.

Such researches have been studied by many research institutes since C. W. Tang et al. of Kodak Co., Ltd. showed that an organic thin-film device emits light at high luminance. The typical structure of an organic thin-film light emitting device proposed by a research group of Kodak Co., Ltd. is such that a hole-transporting diamine compound, an emissive layer made of 8-hydroxyquinoline aluminum, and a cathode made of a Mg : Ag are formed sequentially on an ITO glass substrate, and the device was able to emit green light of 1,000 cd/m² at a driving voltage of about 10 V (see, for example, Non-patent Document 1).

Then, as a result of many researches for practical application, organic thin-film light emitting devices are steadily in practical use, for example, have been adopted for main displays of cellular phones, etc. However, there are still many technical problems and, especially, attainment of both increased efficiency and prolonged life of a device is one of the major problems.

The driving voltage of a device greatly depends on a carrier transporting material that transports carriers such as a hole and an electron to an emissive layer. Materials having a carbazole skeleton are known as materials to transport holes (hole transporting materials) (see, for example, Patent Documents 1 to 4).

### [PRIOR ART DOCUMENTS]

### [PATENT DOCUMENTS]

Patent Document 1: WO 2009/61145
Patent Document 2: WO 2010/41872
Patent Document 3: JP-A-8-3547
Patent Document 4: JP-A-2008-294161

### [NON-PATENT DOCUMENT]

Non-Patent Document 1: Applied Physics Letters (USA), 1987, Vol. 51, No. 12, p. 913

### [SUMMARY OF THE INVENTION]

### [PROBLEMS TO BE SOLVED BY THE INVENTION]

However, conventional technologies were difficult to reduce the driving voltage of a device sufficiently, and even if they had been able to reduce the driving voltage, the luminance efficiency and the durable life of a device were insufficient. Thus, technologies capable of realizing a low driving voltage, a high luminance efficiency, and a durable life have not been found, yet.

An object of the present invention is to solve such problems with the conventional technologies and provide an organic thin-film light emitting device that has improved luminance efficiency and durable life while maintaining a low driving voltage.

### [SOLUTIONS TO THE PROBLEMS]

The present invention is a light emitting device material including a compound represented by the following general formula (1) :

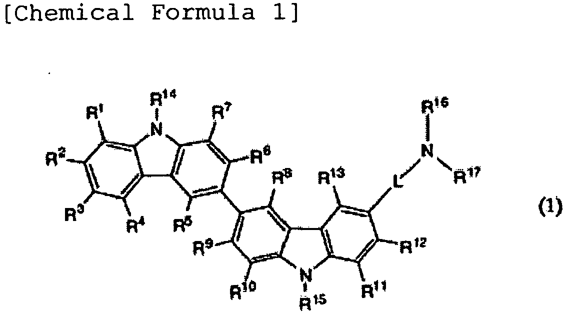

wherein R¹ to R¹³ may be the same or different and are each selected from the group consisting of hydrogen, deuterium, an alkyl group, a cycloalkyl group, an amino group, an aryl group, a heterocyclic group, a heteroaryl group, an alkenyl group, a cycloalkenyl group, an alkynyl group, an alkoxy group, an alkylthio group, an aryl ether group, an aryl thioether group, halogen, a cyano group, a carbonyl group, a carboxyl group, an oxycarbonyl group, a carbamoyl group, -P(=O)R¹⁸R¹⁹, and a silyl group; R¹⁸ and R¹⁹ are each an aryl group or a heteroaryl group; these substituents each may be further substituted and adjoining substituents may further form a ring; R¹⁴ and R¹⁵ may be the same or different and are each selected from the group consisting of an alkyl group, an aryl group, an alkenyl group, an alkylthio group, an arylthio group, a heterocyclic group, and a heteroaryl group; L is an alkylene group, an arylene group, or a heteroarylene group; R¹⁶ and R¹⁷ may be the same or different and are each selected from the group consisting of an alkyl group, an aryl group, an alkenyl group, an alkylthio group, an arylthio group, a heterocyclic group, and a heteroaryl group; and R¹⁴ to R¹⁷ each may be further substituted.

### [EFFECTS OF THE INVENTION]

According to the present invention, there can be provided an organic electric field light emitting device being low in driving voltage, having a high luminance efficiency, and further having a durable life long enough.

### [EMBODIMENTS OF THE INVENTION]

The compound represented by the general formula (1) in the present invention is described in detail below.

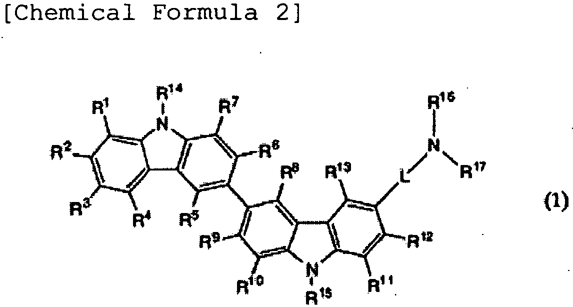

R¹ to R¹³ may be the same or different and are each selected from the group consisting of hydrogen, deuterium, an alkyl group, a cycloalkyl group, an amino group, an aryl group, a heterocyclic group, a heteroaryl group, an alkenyl group, a cycloalkenyl group, an alkynyl group, an alkoxy group, an alkylthio group, an aryl ether group, an aryl thioether group, halogen, a cyano group, a carbonyl group, a carboxyl group, an oxycarbonyl group, a carbamoyl group, -P(=O)R¹⁸R¹⁹, and a silyl group. R¹⁸ and R¹⁹ are each an aryl group or a heteroaryl group. These substituents each may be further substituted and adjoining substituents may further form a ring. R¹⁴ and R¹⁵ may be the same or different and are each selected from the group consisting of an alkyl group, an aryl group, an alkenyl group, an alkylthio group, an arylthio group, a heterocyclic group, and a heteroaryl group. L is an alkylene group, an arylene group, or a heteroarylene group. R¹⁶ and R¹⁷ may be the same or different and are each selected from the group consisting of an alkyl group, an aryl group, an alkenyl group, an alkylthio group, an arylthio group, a heterocyclic group, and a heteroaryl group. R¹⁴ to R¹⁷ each may be further substituted.

Of these substituents, the alkyl group denotes a saturated aliphatic hydrocarbon group, such as a methyl group, an ethyl group, a n-propyl group, an isopropyl group, a n-butyl group, a sec-butyl group, or a tert-butyl group, and it may or may not have a substituent. An additional substituent when substituted is not particularly limited, and examples thereof may include an alkyl group, an aryl group, and a heteroaryl group, and this point is also common to the following descriptions. In addition, the number of carbon atoms in the alkyl group is not particularly limited, but from the viewpoints of easy availability and cost, it is usually within the range of 1 or more and 20 or less, more preferably 1 or more and 8 or less.

The cycloalkyl group denotes a saturated alicyclic hydrocarbon group, such as cyclopropyl, cyclohexyl, norbornyl, and adamantyl, and this may or may not have a substituent. The number of carbon atoms in the alkyl group moiety is not particularly limited, but is usually within the range of 3 or more and 20 or less.

The amino group may or may not have a substituent, and examples of the substituent include an aryl group and a heteroaryl group. Such a substituent may be further substituted.

The aryl group represents an aromatic hydrocarbon group, such as a phenyl group, a naphthyl group, a biphenyl group, a fluorenyl group, a phenanthryl group, an anthracenyl group, a triphenylenyl group, a terphenyl group, and a pyrenyl group. The aryl group may or may not have a substituent. The number of carbon atoms in the aryl group is not particularly limited, but is usually within the range of 6 or more and 40 or less.

The heterocyclic group denotes an aliphatic ring having an atom other than carbon in the ring, such as a pyran ring, a piperidine ring, and a cyclic amide, and this may or may not have a substituent. The number of carbon atoms in the heterocyclic group is not particularly limited, but is usually within the range of 2 or more and 20 or less.

The heteroaryl group denotes a cyclic aromatic group having one or a plurality of atoms other than carbon in the ring, such as a furanyl group, a thiophenyl group, a pyridyl group, a quinolinyl group, a pyrazinyl group, a naphthyridyl group, a benzofuranyl group, a benzothiophenyl group, an indolyl group, a dibenzofuranyl group, a dibenzothiophenyl group, and a carbazolyl group, and this may be unsubstituted or substituted. The number of carbon atoms in the heteroaryl group is not particularly limited, but is usually within the range of 2 or more and 30 or less.

The alkenyl group denotes an unsaturated aliphatic hydrocarbon group containing a double bond, such as a vinyl group, an allyl group, and a butadienyl group, and this may or may not have a substituent. The number of carbon atoms in the alkenyl group is not particularly limited, but is usually within the range of 2 or more and 20 or less.

The cycloalkenyl group denotes an unsaturated alicyclic hydrocarbon group containing a double bond, such as a cyclopentenyl group, a cyclopentadienyl group, and a cyclohexenyl group, and this may or may not have a substituent. The number of carbon atoms of the cycloalkenyl group is not specifically limited, but is usually within the range of 2 or more and 20 or less.

The alkynyl group denotes an unsaturated aliphatic hydrocarbon group containing a triple bond, such as an ethynyl group, and this may or may not have a substituent. The number of carbon atoms in the alkynyl group is not particularly limited, but is usually within the range of 2 or more and 20 or less.

The alkoxy group denotes a functional group in which an aliphatic hydrocarbon group is bound via an ether bond, such as a methoxy group, an ethoxy group and a propoxy group, and this aliphatic hydrocarbon group may or may not have a substituent. The number of carbon atoms in the alkoxy group is not particularly limited, but is usually within the range of 1 or more and 20 or less.

The alkylthio group is a group in which the oxygen atom of an ether bond of the alkoxy group is substituted with a sulfur atom. The hydrocarbon group of the alkylthio group may or may not have a substituent. The number of carbon atoms in the alkylthio group is not particularly limited, but is usually within the range of 1 or more and 20 or less.

The aryl ether group denotes a functional group in which an aromatic hydrocarbon group is bound via an ether bond, such as a phenoxy group, and the aromatic hydrocarbon group may or may not have a substituent. The number of carbon atoms in the aryl ether group is not particularly limited, but is usually within the range of 6 or more and 40 or less.

The aryl thioether group is a group in which the oxygen atom of an ether bond of the aryl ether group is substituted with a sulfur atom. The aromatic hydrocarbon group in the aryl ether group may or may not have a substituent. The number of carbon atoms in the aryl ether group is not particularly limited, but is usually within the range of 6 or more and 40 or less.

The halogen denotes fluorine, chlorine, bromine or iodine.

The carbonyl group, the carboxyl group, the oxycarbonyl group, and the carbamoyl group may or may not have a substituent, and examples of the substituent include an alkyl group, a cycloalkyl group and an aryl group, and these substituents may be further substituted.

The silyl group denotes a functional group having a bond with a silicon atom, such as a trimethylsilyl group, and this may or may not have a substituent. The number of carbon atoms in the silyl group is not particularly limited, but is usually within the range of 3 or more and 20 or less. In addition, the number of silicon is usually within the range of 1 or more and 6 or less.

The alkylene group denotes a divalent group derived from an alkyl group, and examples thereof include a methylene group, an ethylene group, a n-propylene group, an isopropylene group, a n-butylene group, a sec-butylene group, and a tert-butylene group. These substituents each may or may not have a substituent. The number of carbon atoms in the alkylene group is not particularly limited, but is usually within the range of 1 or more and 20 or less.

The arylene group denotes a divalent group derived from an aryl group, and examples thereof include a phenylene group, a naphthylene group, a biphenylene group, a fluorenylene group, a phenanthrylene group, a terphenylene group, an anthracenylene group, and a pyrenylene group. These substituents each may or may not have a substituent. The number of carbon atoms in the arylene group is not particularly limited, but is usually within the range of 6 or more and 40 or less. When the arylene group has a substituent, the number of carbon atoms including those of the substituent is preferably within the range of 6 or more and 60 or less.

The heteroarylene group denotes a divalent group derived from a cyclic aromatic group having one or a plurality of atoms other than carbon in the ring, and examples thereof include a furanylene group, a thiophenylene group, a pyridylene group, a quinolinylene group, an isoquinolinylene group, a pyrazinylene group, a pyrimidylene group, a naphthylidylene group, a benzofuranylene group, a benzothiophenylene group, an indolylene group, a dibenzofuranylene group, a dibenzothiophenylene group, and a carbazolylene group. These substituents each may or may not have a substituent. The number of carbon atoms in the heteroarylene group is not particularly limited, but is usually within the range of 2 or more and 30 or less. When the heteroarylene group has a substituent, the number of carbon atoms including those of the substituent is preferably within the range of 2 more and 50 or less.

The compound represented by the general formula (1) is preferably an asymmetric carbazole dimer from the viewpoint of the improvement in the durability of a device. This is because in a symmetrical structure like that disclosed in Patent Document 4, the durability of a device drops because of lack of the stability of a thin film due to high crystallinity. In the present invention, the asymmetric carbazole dimer denotes one of general formula (1) wherein R³ is a group other than the group represented by LNR¹⁶R¹⁷. Preferably, R³ is hydrogen, deuterium, an alkyl group, a cycloalkyl group, an alkoxy group, an unsubstituted aryl group, or an unsubstituted heteroaryl group.

Considering the easy availability of raw materials and the synthesis cost, it is preferable that all the groups R¹ to R¹³ are groups selected from hydrogen and deuterium or that R³ is a group other than hydrogen and deuterium and the rest are groups selected from hydrogen and deuterium. When R³ is a group other than hydrogen and deuterium, R³ is preferably an alkyl group, a cycloalkyl group, an alkoxy group, an aryl group, or a heteroaryl group. These groups each may be further substituted. Of these groups, preferred is an alkyl group, an alkoxy group, or an aryl group. It is also preferable that R³ is an N-substituted carbazolyl group, such as an N-phenylcarbazolyl group and, accordingly, the compound represented by the general formula (1) is a carbazole trimer. In this case, the N-phenylcarbazolyl group may be further substituted with an alkyl group, a cycloalkyl group, an alkoxy group, an aryl group, or a heteroaryl group.

Considering the thermal stability or the electrochemical stability of a material, R¹⁴ and R¹⁵ are each preferably a substituted or unsubstituted aryl or heteroaryl group. Examples of the substituent in the case where these groups are substituted include deuterium, an alkyl group, an alkoxy group, an aryl group, a heteroaryl group, and an amino group. Moreover, R¹⁴ and R¹⁵ are each preferably an aryl group having 6 to 30 nucleus carbon atoms or a heteroaryl group having 2 to 30 nucleus carbon atoms. This is because there is a fear of heat decomposition at the time of evaporation if the molecular weights of R¹⁴ and R¹⁵ are excessively large. Of these, more preferred is a phenyl group, a biphenyl group, a fluorenyl group, a dibenzofuranyl group, or a dibenzothiophenyl group. Moreover, from the viewpoint of not reducing a triplet level, an unsubstituted phenyl group or an alkyl-substituted phenyl group is more preferred, and an unsubstituted phenyl group is particularly preferred.

Considering the easy availability of raw materials and the synthesis cost, L is preferably a substituted or unsubstituted, arylene or heteroarylene group. Examples of a preferable substituent when these groups are substituted include deuterium, a methyl group, and a methoxy group. Moreover, L is preferably an arylene group having 6 to 30 nucleus carbon atoms or a heteroarylene group having 2 to 30 nucleus carbon atoms. This is because there is a fear of heat decomposition at the time of evaporation if the molecular weight of L is excessively large. Of these, more preferred is a phenylene group, a biphenylene group, a fluorenylene group, a furanylene group, a thienylene group, a dibenzofuranylene group, or a dibenzothiophenylene group. Especially, a substituted or unsubstituted, phenylene, biphenylene, or fluorenylene group is particularly preferable from the viewpoint of failing to lengthen conjugation greatly and not reducing a triplet level.

Considering the thermal stability or the electrochemical stability of a material, R¹⁶ and R¹⁷ are each preferably a substituted or unsubstituted aryl or heteroaryl group. Examples of the substituent in the case where these groups are substituted include deuterium, an alkyl group, an alkoxy group, an aryl group, a heteroaryl group, and an amino group. Moreover, R¹⁶ and R¹⁷ are each preferably an aryl group having 2 to 30 nucleus carbon atoms or a heteroaryl group having 2 to 30 nucleus carbon atoms. This is because there is a fear of heat decomposition at the time of evaporation if the molecular weights of R¹⁶ and R¹⁷ are excessively large. Of these, more preferred is a phenyl group, a biphenyl group, a fluorenyl group, a dibenzofuranyl group, or a dibenzothiophenyl group. Moreover, an unsubstituted phenyl group, an alkyl-substituted phenyl group, a biphenyl group, or a fluorenyl group is particularly preferable from the viewpoint of not reducing a triplet level.

The combination of L, R¹⁶, and R¹⁷ is not particularly limited, and specific examples thereof include the following skeletons.

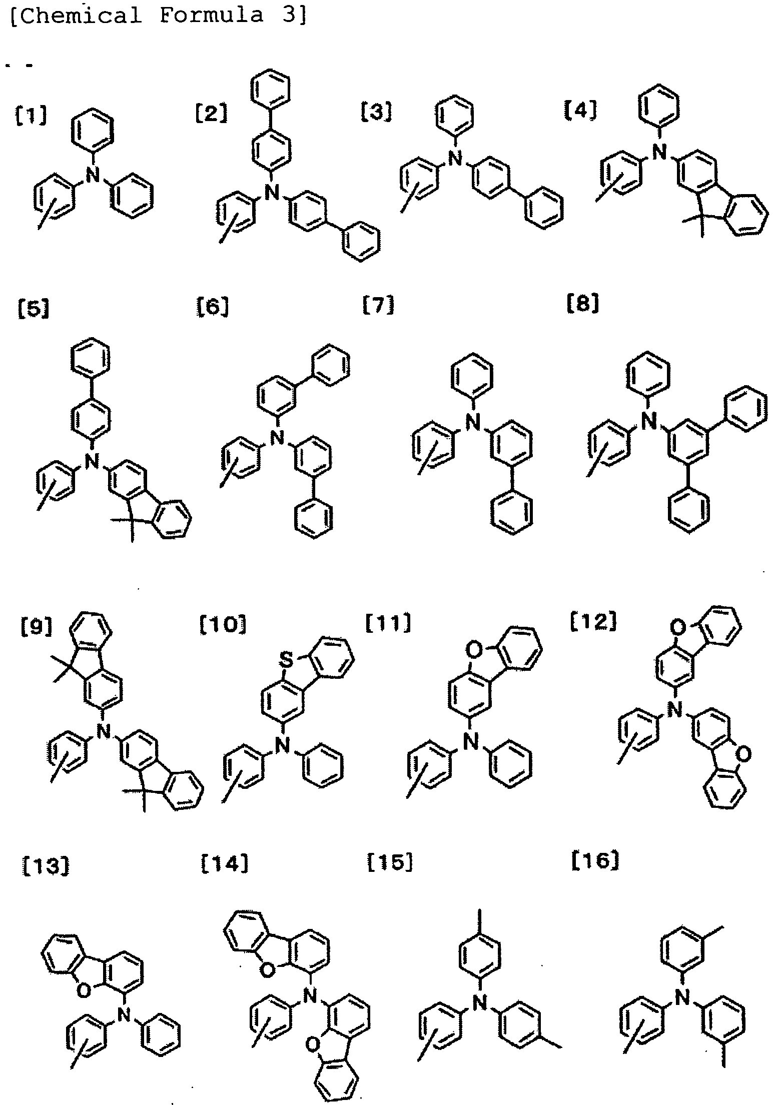

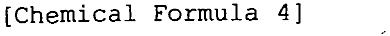

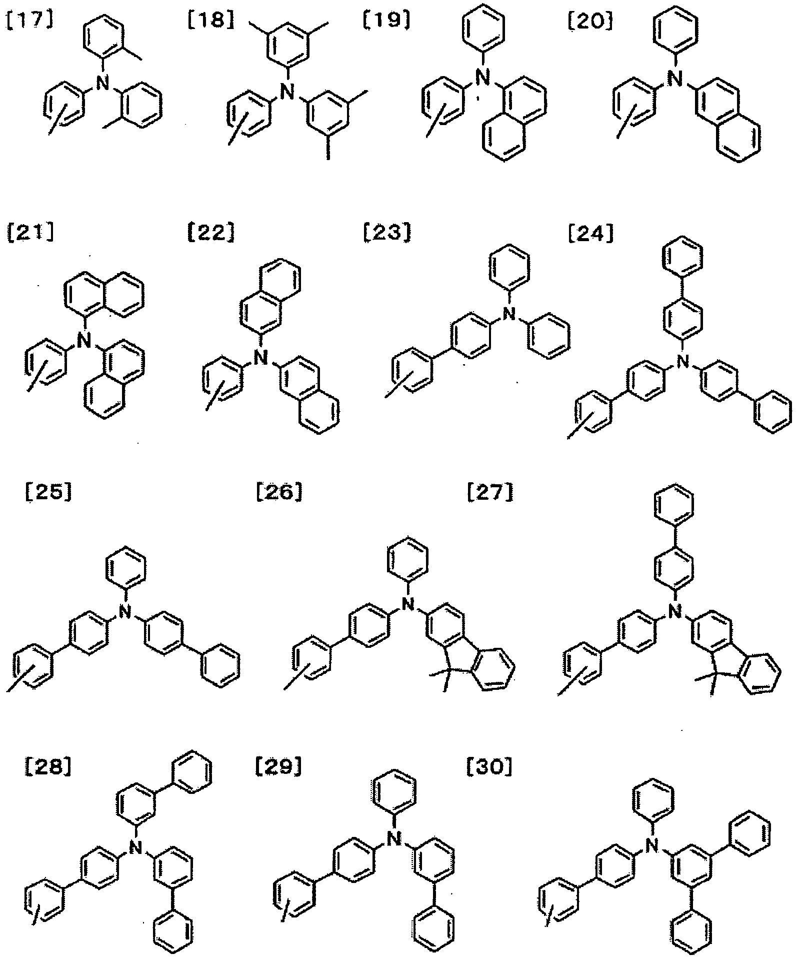

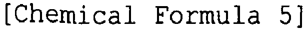

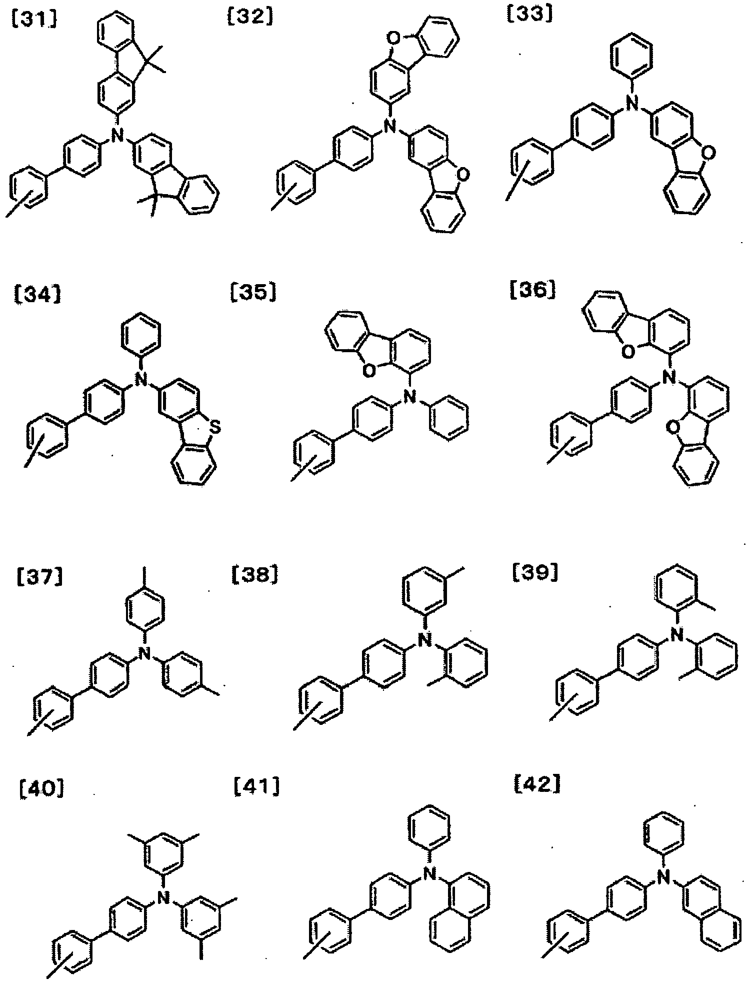

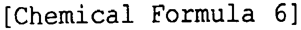

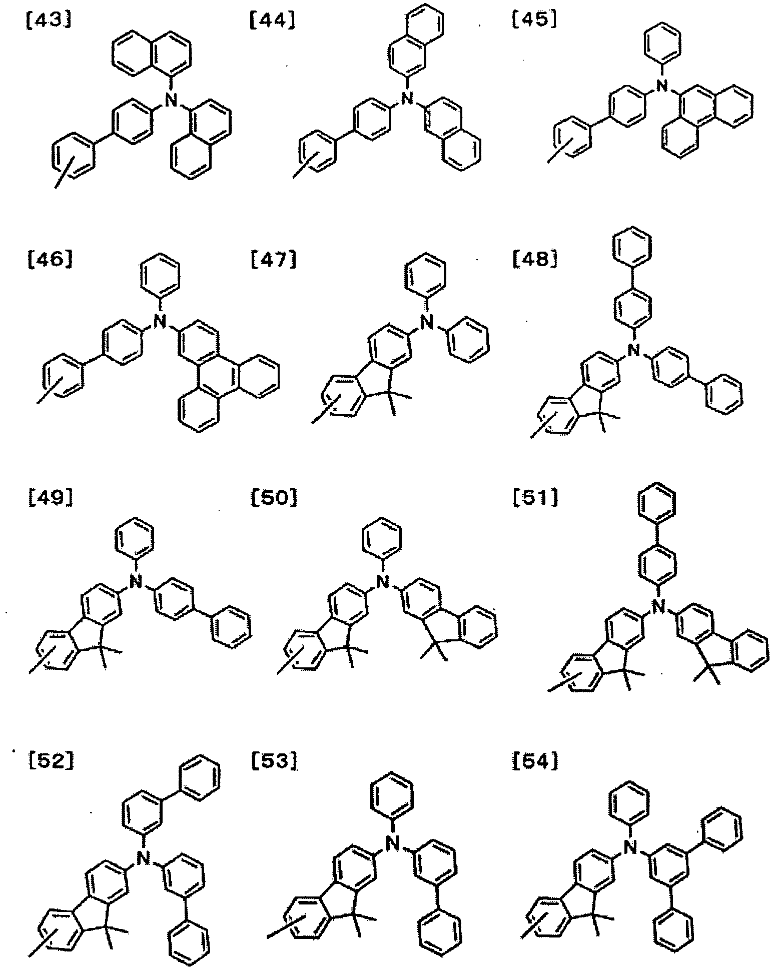

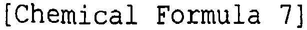

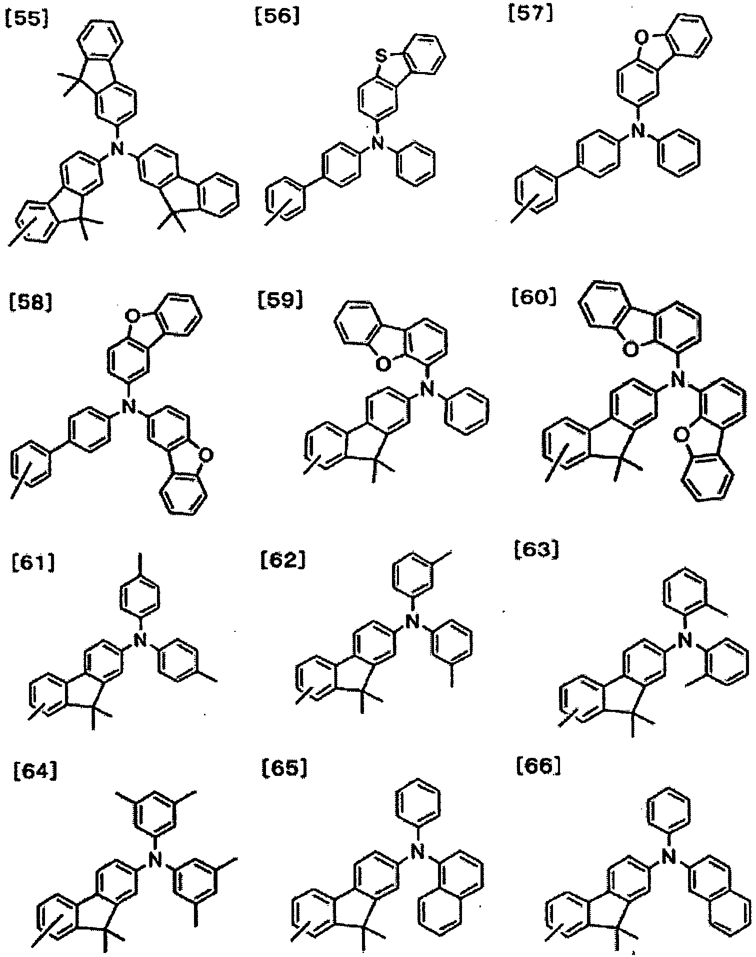

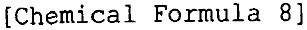

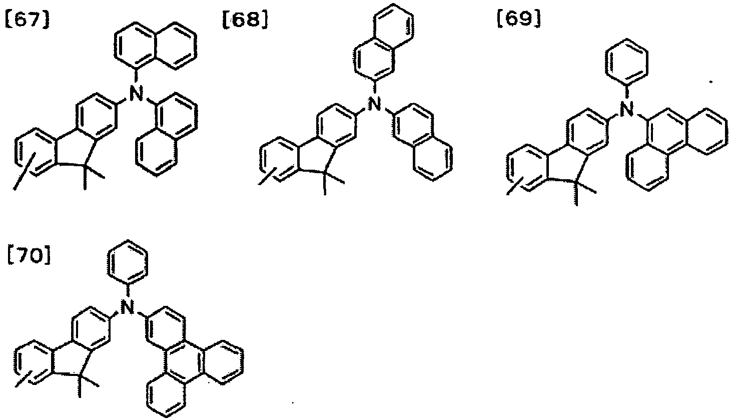

Specific examples of such compounds represented by the general formula (1) include the following compounds. The examples of the compound represented by the general formula (1) are not restricted to these, and for example, compounds formed by arbitrarily combining the combinations of L, R¹⁶, and R¹⁷ provided above with the carbazole dimer or trimer structure in the compounds provided below as examples are also to be preferably used.

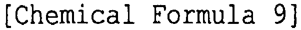

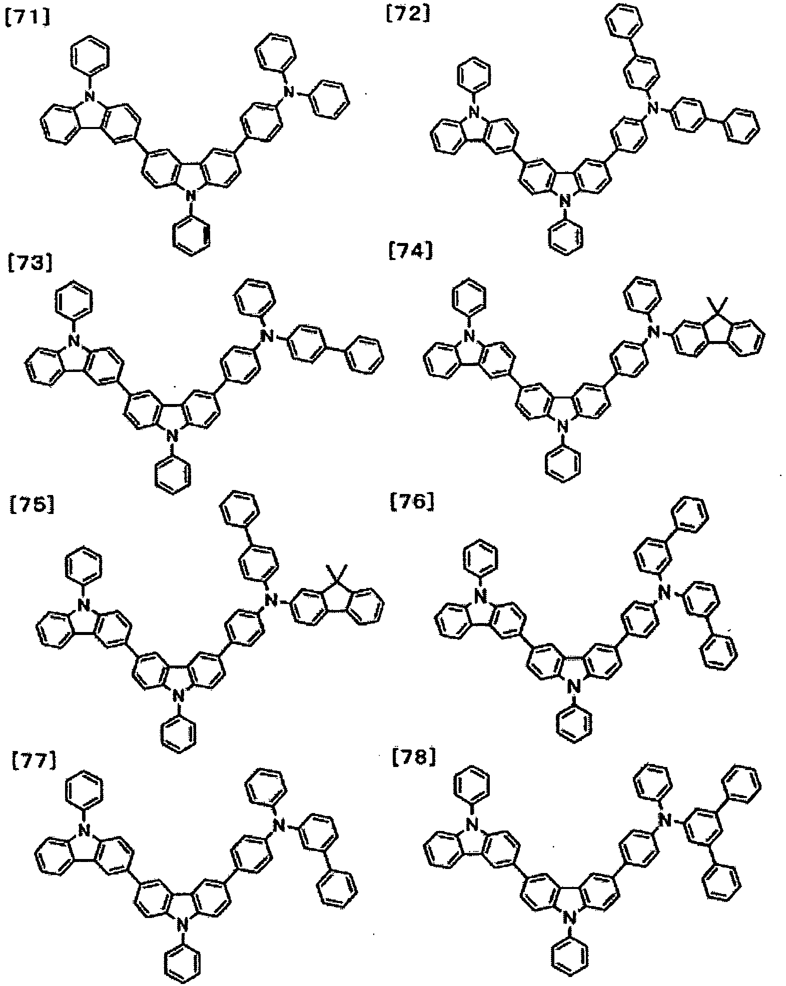

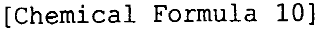

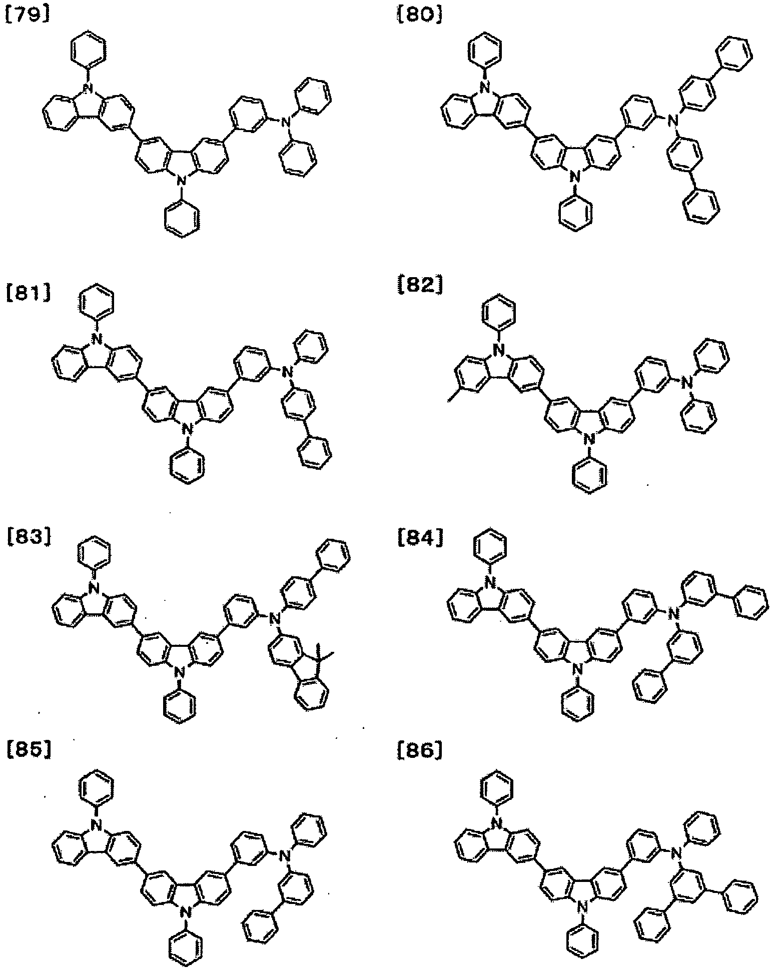

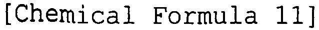

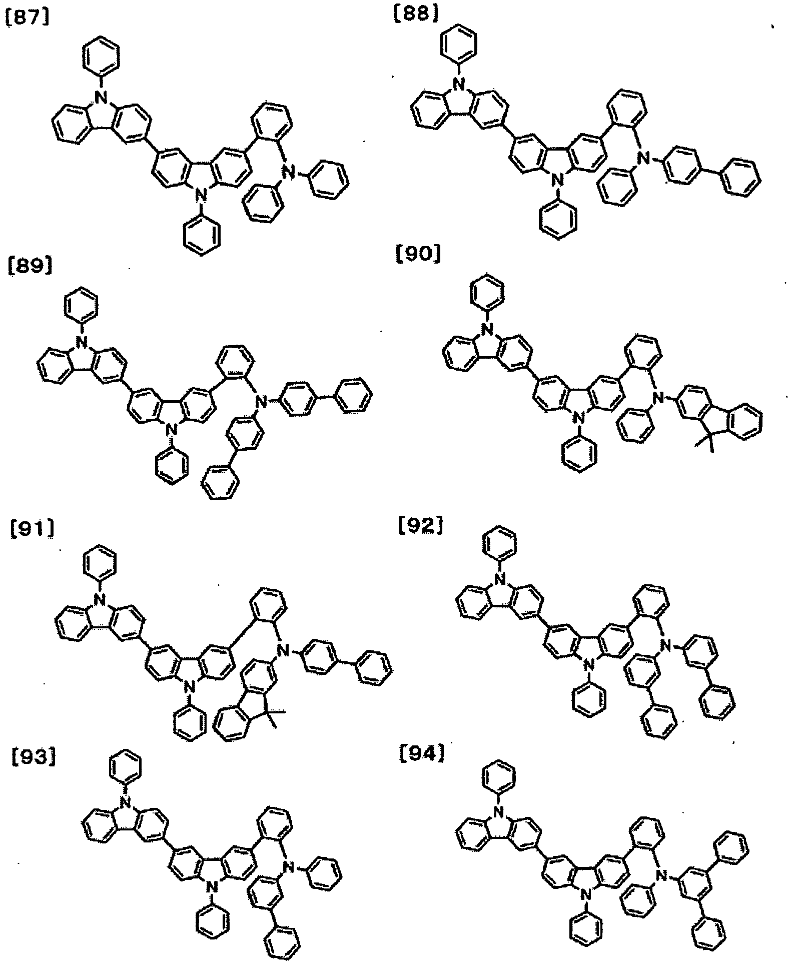

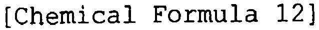

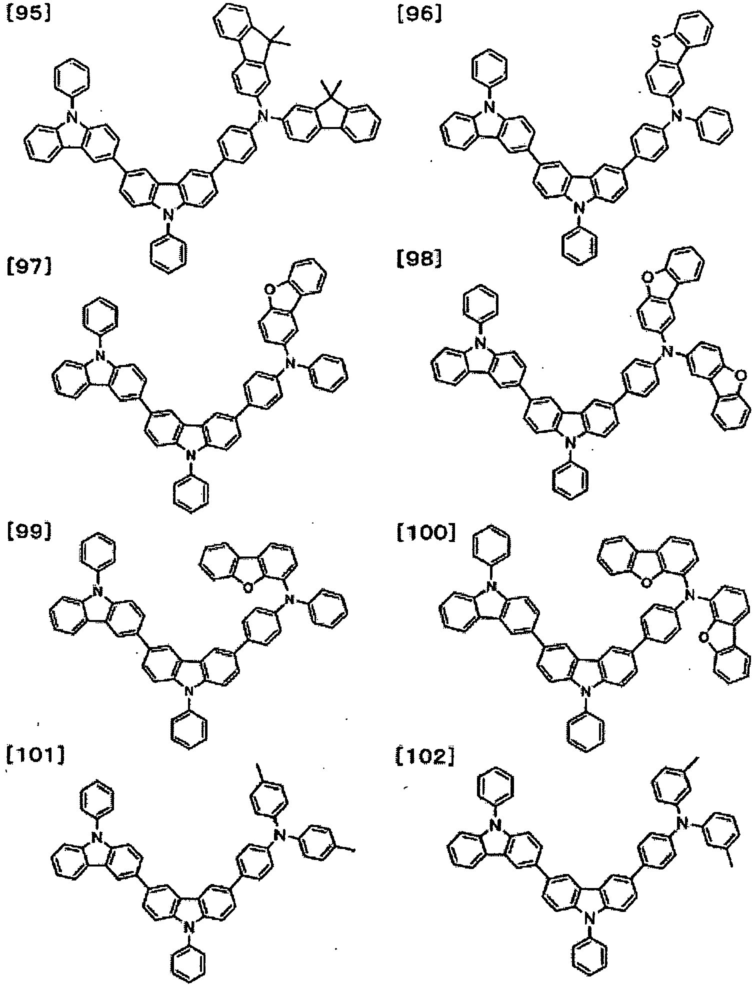

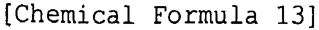

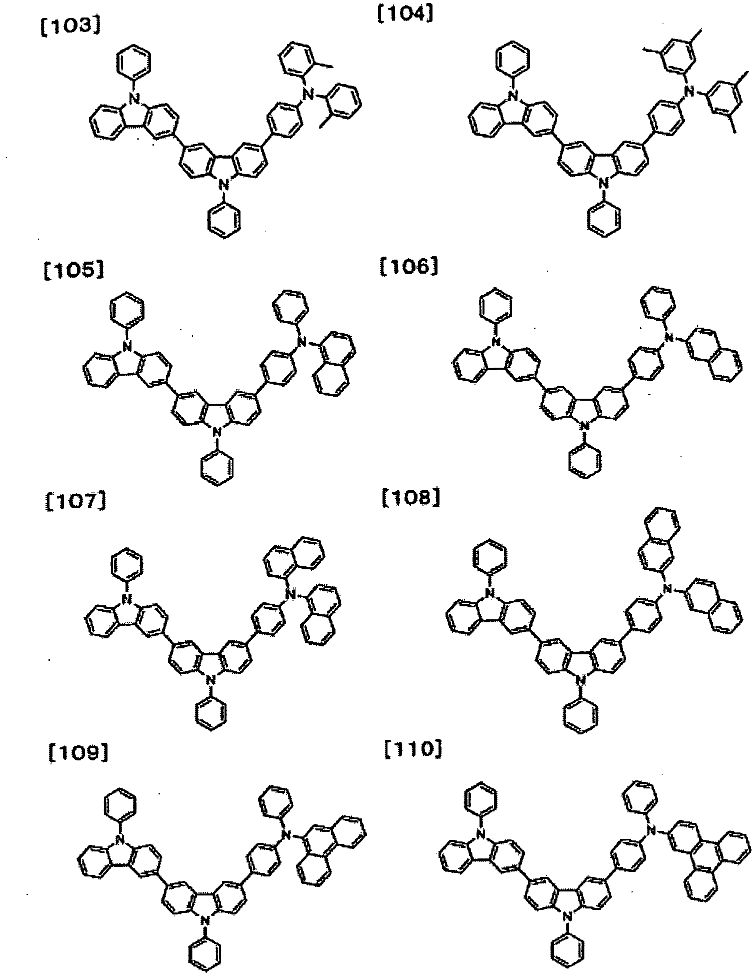

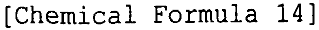

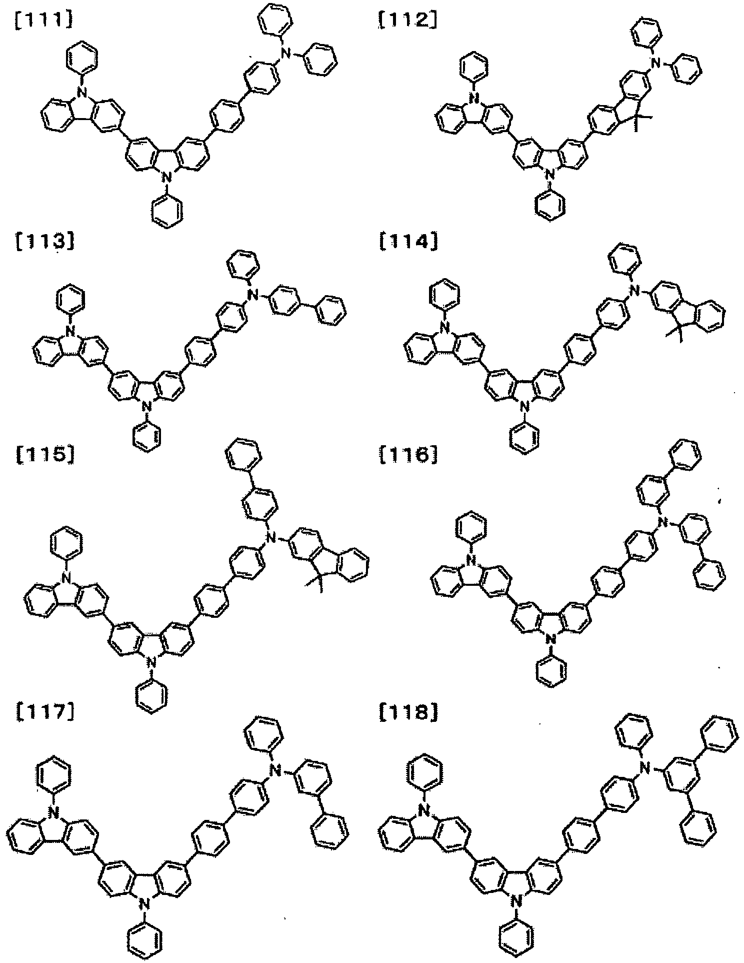

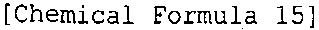

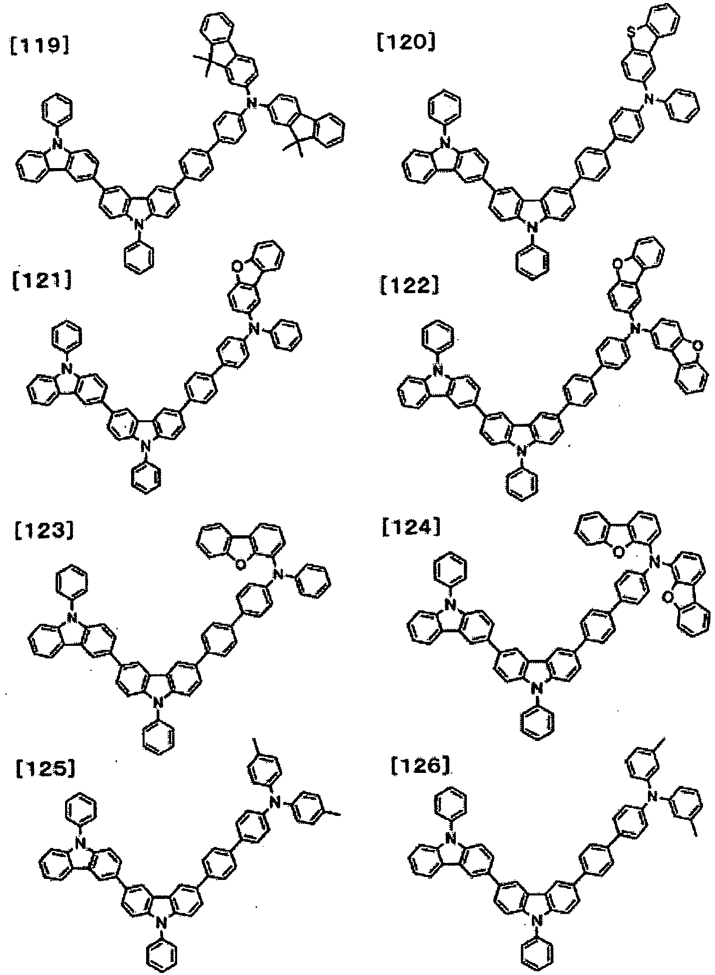

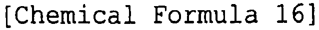

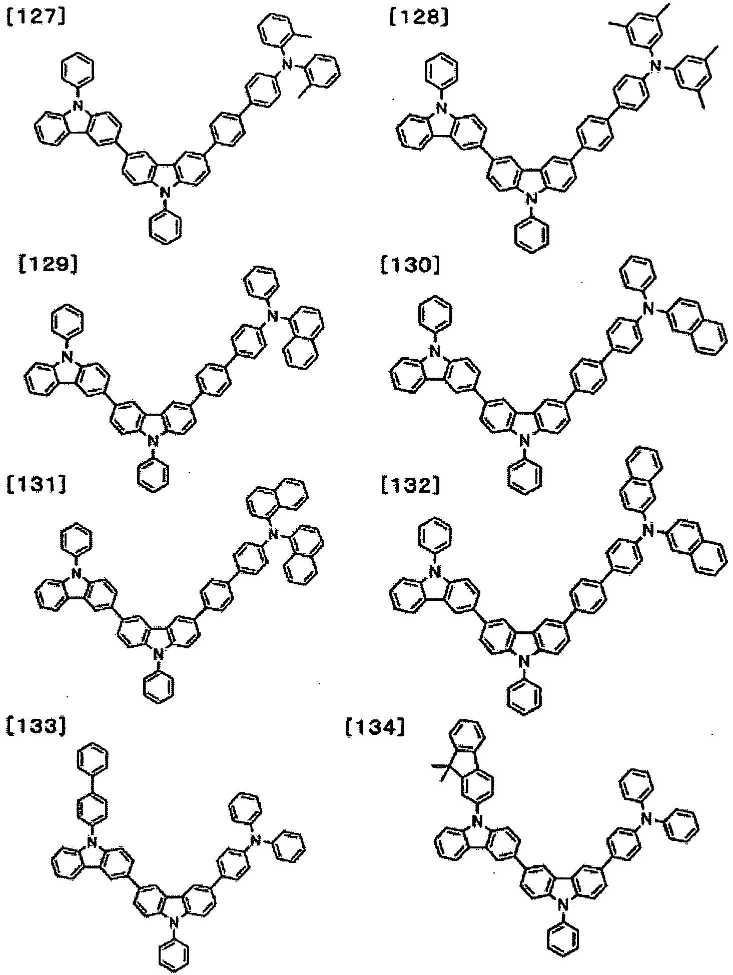

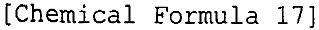

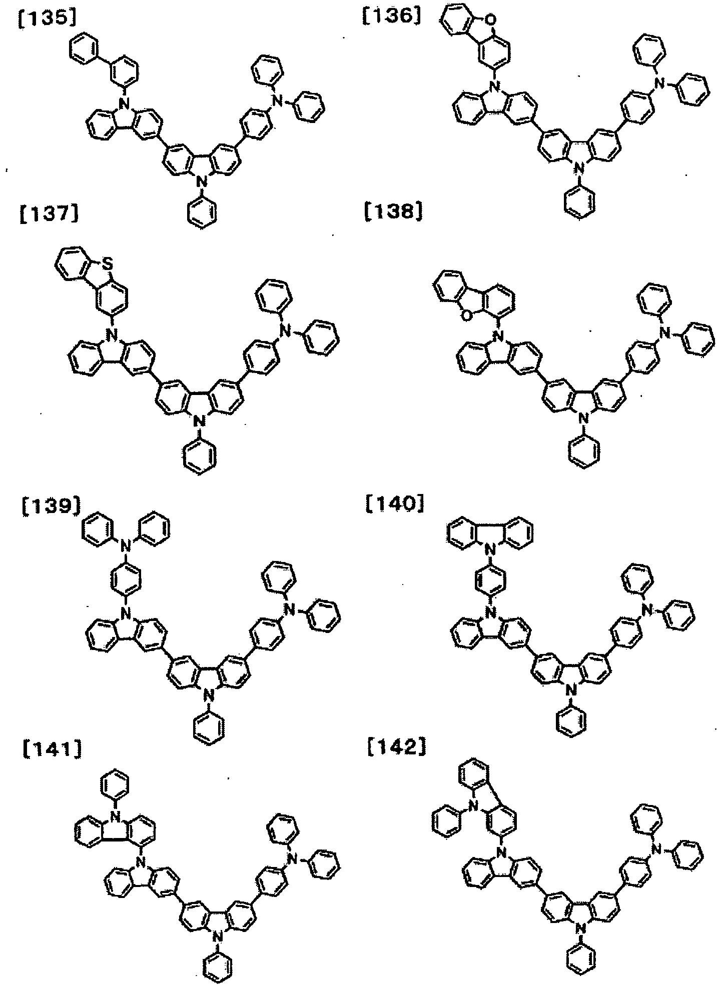

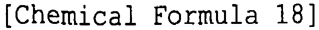

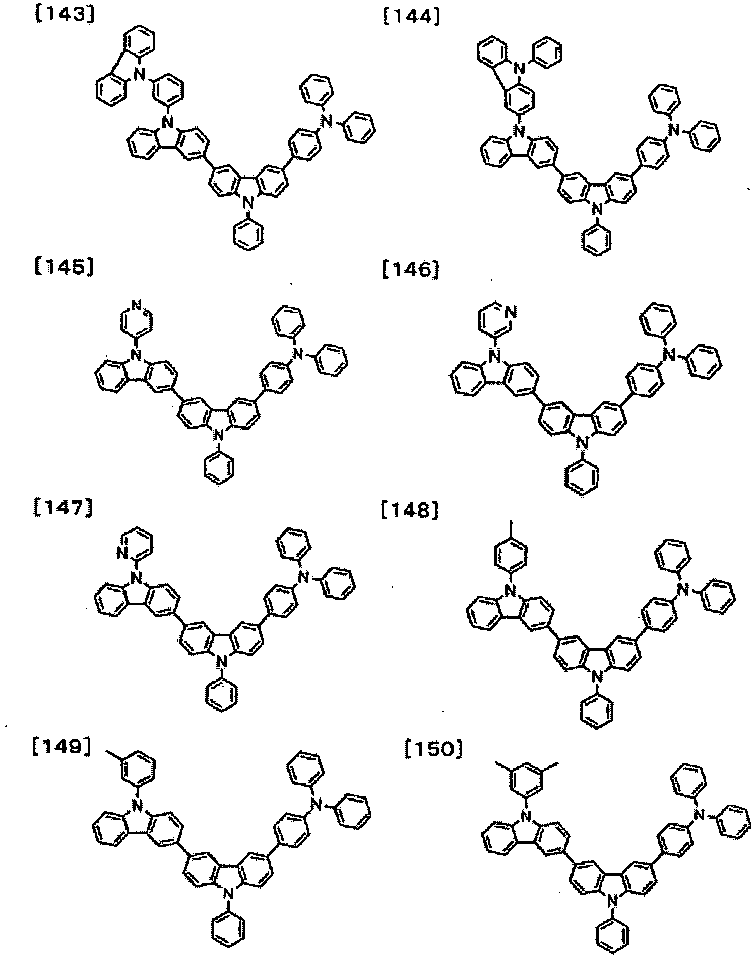

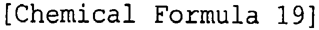

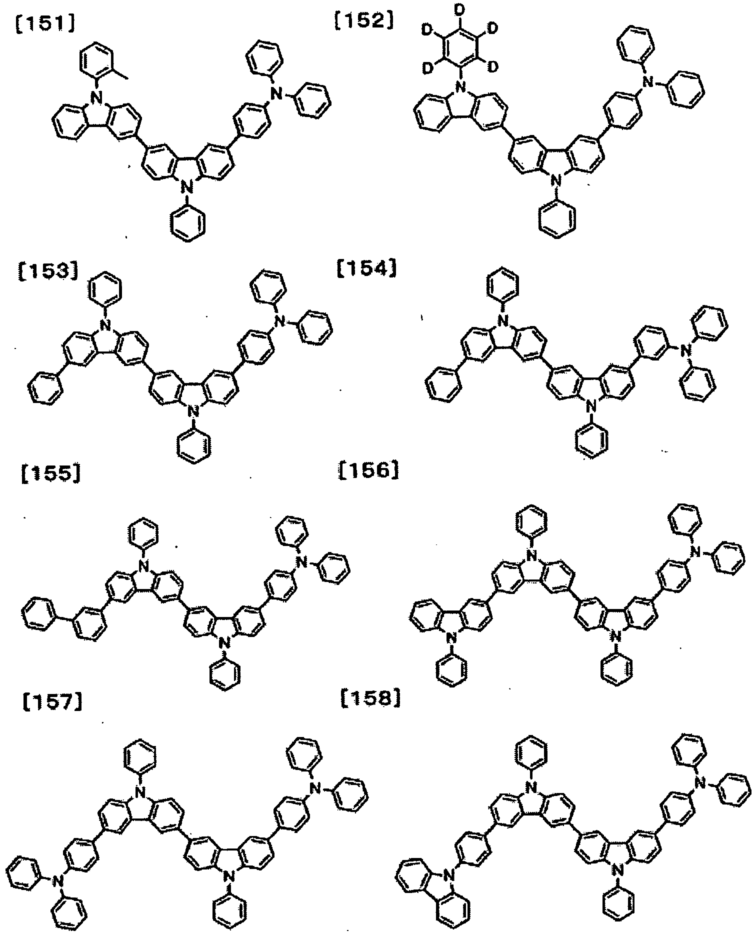

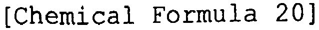

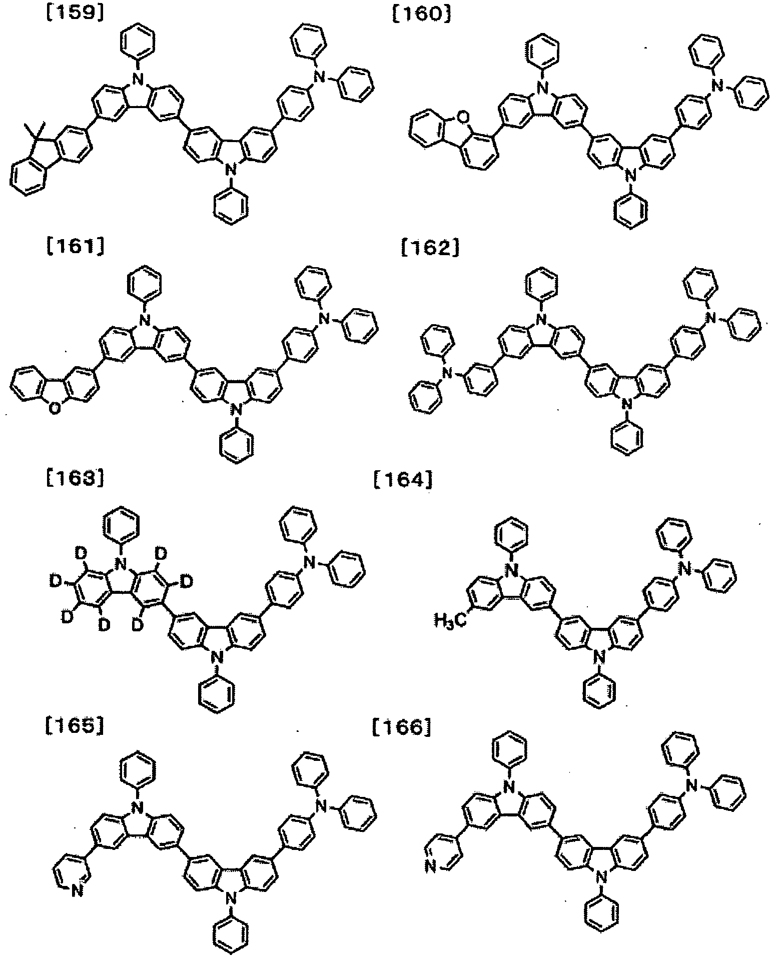

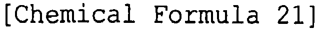

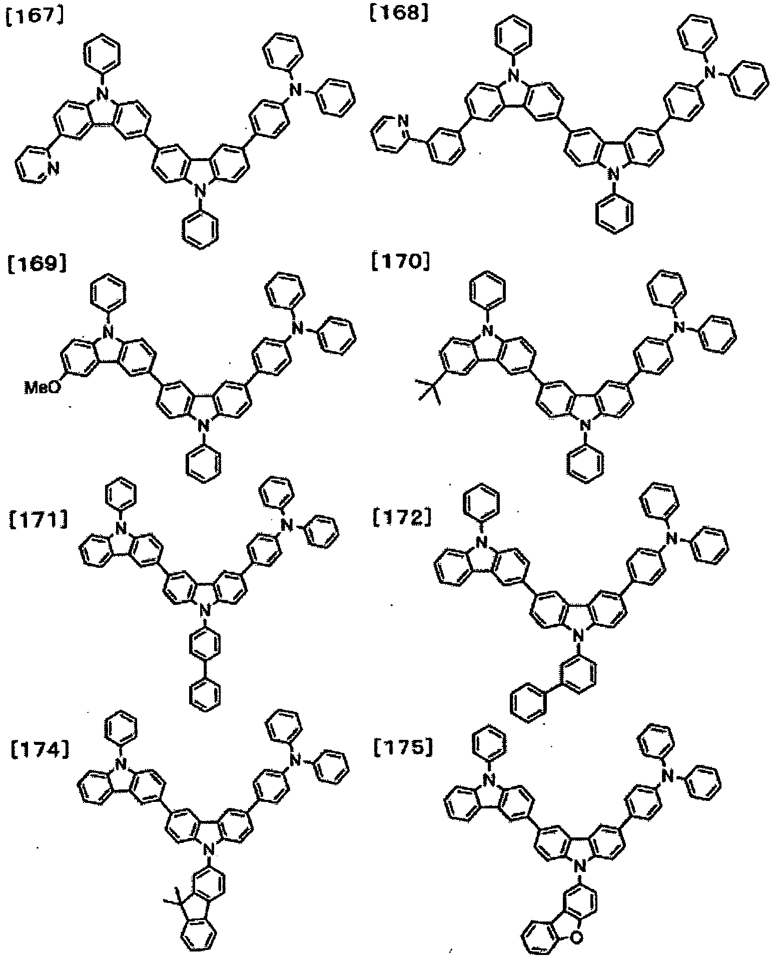

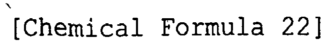

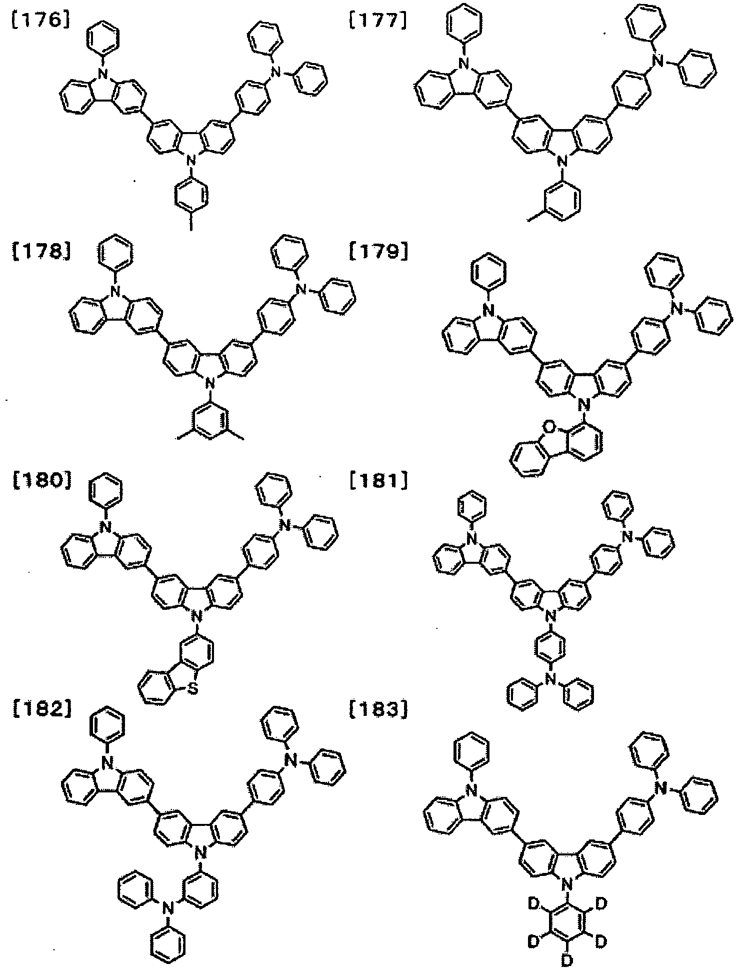

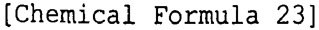

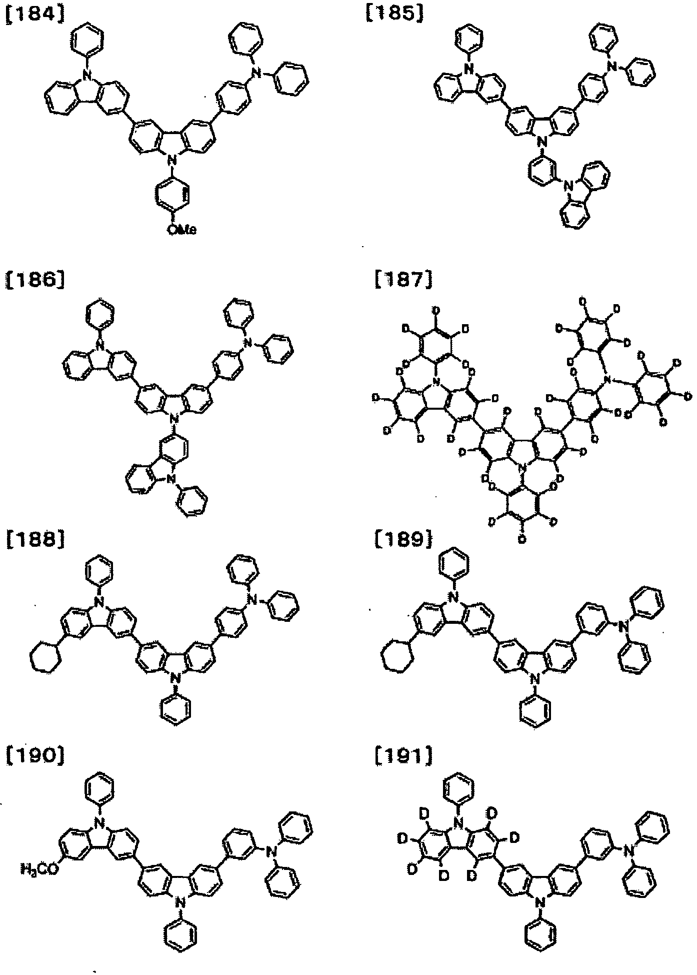

The compound represented by the general formula (1) can be produced by conventional methods. For example, a monobromo form of a carbazole dimer is synthesized first via a Suzuki coupling reaction of a dibromo form of a 9-substituted carbazole and a monoboronic acid of a 9-substituted carbazole. On the other hand, the compound represented by the general formula (1) can be easily synthesized by converting a monobromo form of a triarylamine into a monoboronic acid ester form and then performing a Suzuki coupling reaction with the aforementioned monobromo form of a carbazole dimer, but the production method is not limited to this.

The compound represented by the general formula (1) in the present invention is used as a light emitting device material. Herein, the light emitting device material in the present invention denotes a material to be used in any layer of a light emitting device and also includes a material to be used in a protective film of a cathode, in addition to materials to be used in a hole injection layer, a hole transporting layer, an emissive layer and/or an electron transporting layer as described later. Use of the compound represented by the general formula (1) in the present invention in any layer of a light emitting device can afford a high luminance efficiency and also can afford a light emitting device superior in durability.

Next, embodiments of the light emitting device of the present invention will be described in detail. The light emitting device of the present invention has an anode and a cathode, and an organic layer interposing between the anode and the cathode, and the organic layer emits light by electric energy.

The layer configuration between the anode and the cathode in such a light emitting device include, besides configurations made up of only an emissive layer, laminated configurations such as 1) emissive layer/electron transporting layer, 2) hole transporting layer/emissive layer, 3) hole transporting layer/emissive layer/electron transporting layer, 4) hole injection layer/hole transporting layer/emissive layer/electron transporting layer, 5) hole transporting layer/emissive layer/electron transporting layer/electron injection layer, and 6) hole injection layer/hole transporting layer/emissive layer/electron transporting layer/electron injection layer. Each of the layers may be in the form of a single layer or a plurality of layers.

While the compound represented by the general formula (1) can be used for any layer of the layers described above in a light emitting device, it is particularly suitably used for a hole transporting layer.

In the light emitting device of the present invention, the anode and the cathode have a role for supplying a sufficient current for light emission of the device, and it is desirable that at least one of them is transparent or translucent in order to take out light. Usually, the anode formed on a substrate is made to be a transparent electrode.

While the material to be used for an anode is not particularly limited and may be electrically conductive metal oxides, such as zinc oxide, tin oxide, indium oxide, tin oxide indium (ITO), and zinc-oxide indium (IZO), metals, such as gold, silver, and chromium, inorganic electrically conductive substances, such as copper iodide and copper sulfide, electrically conductive polymers, such as polythiophene, polypyrrole, and polyaniline as long as being a material that is capable of injecting holes into an organic layer efficiently and that is transparent or translucent in order to take out light, use of ITO glass or NESA glass is particularly desirable. These electrode materials may be used alone, or a plurality of materials may be used in lamination or in admixture. Since it is favorable that a sufficient current for light emission of the device can be supplied, the resistance of a transparent electrode is not limited, but from the viewpoint of the power consumption of the device, a low resistance is desirable. For example, an ITO substrate having 300 Ω/□ or lower functions as a device electrode, but since currently, it is possible to supply a current to a substrate having around 10 Ω/□, it is particularly desirable to use a substrate having a low resistance of 20 Ω/□ or lower. The thickness of ITO can be arbitrarily selected according to a resistance value, but ITO is usually used at a thickness of between 50 to 300 nm in many cases.

In addition, in order to retain the mechanical strength of the light emitting device, it is preferable to form the light emitting device on a substrate. As the substrate, a glass substrate such as soda glass or alkali-free glass is suitably used. Since it is favorable that the thickness of a glass substrate has a sufficient thickness for retaining the mechanical strength, 0.5 mm or more is sufficient. Regarding the material of glass, since it is preferable that the amount of ions eluted from glass is low, alkali-free glass is preferable. Alternatively, since soda lime glass provided with a barrier coating such as SiO₂ is commercially available, it can also be used. Further, as far as the first electrode stably functions, it is not necessary that the substrate is glass and, for example, the anode may be formed on a plastic substrate. Examples of a method of forming an ITO film include, but are not particularly limited to, an electron beam method, a sputtering method, and a chemical reaction method.

A material used in the cathode is not particularly limited, as far as it is a substance which can efficiently inject electrons into the emissive layer. Generally, metals such as platinum, gold, silver, copper, iron, tin, aluminum, and indium, or alloys or multilayer lamination of these metals with metals having a low work function such as lithium, sodium, potassium, calcium and magnesium are preferable. Among them, as a main component, aluminum, silver, and magnesium are preferable from the viewpoints of electric resistance value, easiness of making a film, stability of a film, and luminance efficiency. Particularly, when the material is constituted by magnesium and silver, electron injection into the electron transporting layer and the electron injection layer in the present invention becomes easy, and low voltage driving becomes possible, and therefore it is preferable.

Further, preferable examples include lamination of metals such as platinum, gold, silver, copper, iron, tin, aluminum, and indium, or alloys using these metals, inorganic substances such as silica, titania, and silicon nitride, and organic polymer compounds such as polyvinyl alcohol, polyvinyl chloride, and a hydrocarbon-based polymer compound as a protective film layer on the cathode for protecting the cathode. However, in the case of a device structure for taking out light from the cathode side (top emission structure), the protective film layer is selected from materials having light permeability in a visible light region. Examples of a method of manufacturing these electrodes include, but are not particularly limited to, resistance heating, electron beam, sputtering, ion plating and coating.

The hole injection layer is a layer that is to be inserted to between an anode and a hole transporting layer. A single hole injection layer may be formed or, alternatively, a plurality of hole injection layers may be laminated. It is preferable that the hole injection layer is present between a hole transporting layer and an anode because this successfully results in lower voltage driving, increased durable life, and improvement in luminance efficiency due to improvement in the carrier balance of a device.

While the material to be used for the hole injection layer is not particularly limited, for example, benzidine derivatives. such as 4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB), and bis(N,N'-diphenyl-4-aminophenyl)-N,N-diphenyl-4,4'-diamino-1,1'-biphenyl (TPD232), materials called starburst arylamines, such as 4,4',4"-tris(3-methylphenyl (phenyl)amino)triphenylamine (m-MTDATA) and 4,4',4"-tris(1-naphthyl (phenyl)amino)triphenylamine (1-TNATA), biscarbazole derivatives such as bis(N-arylcarbazole) or bis(N-alkylcarbazole), heterocyclic compounds, such as pyrazoline derivatives, stilbene-based compounds, hydrazone-based compounds, benzofuran derivatives, thiophene derivatives, oxadiazole derivatives, phthalocyanine derivatives, and porphyrin derivatives, and such polymers as polycarbonates styrene derivatives having the above-mentioned monomers on their side chains, polythiophene, polyaniline, polyfluorene, polyvinylcarbazole, and polysilane are used. The compound represented by the general formula (1) may also be used. Especially, benzidine derivatives and starburst arylamine materials are more preferably used from the viewpoint of having a lower HOMO level than the compound represented by the general formula (1) and injecting and transporting holes smoothly from an anode to a hole transporting layer.

Such materials may be used single, or alternatively two or more materials may be used in admixture. A hole injection layer may be formed by laminating a plurality of materials. Moreover, it is more preferable that the hole injection layer is formed of an acceptor compound alone or the hole injection material described above is used with the material doped with an acceptor compound because if so, the effects described above will be remarkably obtained. The acceptor compound is a material that forms a charge transfer complex with a hole transporting layer in contact therewith in the case where the compound is used in the form of a single layer film or forms a charge transfer complex with a material that constitutes a hole injection layer in the case where the compound is used while being doped into the material. Use of such a material improves the electrical conductivity of a hole injection layer and contributes more to drop the driving voltage of a device, thereby affording effects such as improvement in luminance efficiency and improvement in durable life.

Examples of the acceptor compound include metal chlorides such as iron (III) chloride, aluminum chloride, gallium chloride, indium chloride, and antimony chloride, metal oxides such as molybdenum oxide, vanadium oxide, tungsten oxide, and ruthenium oxide, and charge transfer complexes such as tris(4-bromophenyl)aminium hexachloroantimonate (TBPAH). Moreover, organic compounds having a nitro group, a cyano group, halogen, or a trifluoromethyl group in the molecule, quinone-based compounds, acid anhydride-based compounds, and fullerenes can also be used suitably. Specific examples of such compounds include hexacyanobutadiene, hexacyanobenzene, tetracyanoethylene, tetracyanoquinodimethane (TCQM), tetrafluorotetracyanoquinodimethane (F4-TCNQ), p-fluoranil, p-chloranil, p-bromanil, p-benzoquinone, 2,6-dichlorobenzoquinone, 2,5-dichlorobenzoquinone, tetramethylbenzoquinone, 1,2,4,5-tetracyanobenzene, o-dicyanobenzene, p-dicyanobenzene, 1,4-dicyanotetrafluorobenzene, 2,3-dichloro-5,6-dicyanobenzoquinone, p-dinitrobenzene, m-dinitrobenzene, o-dinitrobenzene, p-cyanonitrobenzene, m-cyanonitrobenzene, o-cyanonitrobenzene, 1,4-naphthoquinone, 2,3-dichloronaphthoquinone, 1-nitronaphthalene, 2-nitronaphthalene, 1,3-dinitronaphthalene, 1,5-dinitronaphthalene, 9-cyanoanthracene, 9-nitroanthracene, 9,10-anthraquinone, 1,3,6,8-tetranitrocarbazole, 2,4,7-trinitro-9-fluorenone, 2,3,5,6-tetracyanopyridine, maleic anhydride, phthalic anhydride, C60, and C70.

Of these, metal oxides and cyano group-containing compounds are preferable because they can be handled and evaporated easily and therefore the above-described effects can be obtained easily. In either of the case where a hole injection layer is formed of an acceptor compound alone or the case where a hole injection layer is doped with an acceptor compound, the hole injection layer may be a single layer or may be formed of a plurality of layers laminated.

The hole transporting layer is a layer that transports to an emissive layer holes injected from an anode. The hole transporting layer may be formed of either a single layer or a plurality of layers laminated.

The compound represented by the general formula (1) is preferably used for a hole injection layer and hole transporting layer of a light emitting device because the compound has an ionization potential of 5.3 to 5.5 eV (value of an evaporated film measured using AC-2 (manufactured by RIKEN KEIKI Co., Ltd.)), a high triplet level, high hole transporting property, and high film stability. The compound represented by the general formula (1) has a higher LUMO level and is superior in electron blocking property compared with conventional hole transporting materials having a benzidine skeleton because the compound has a greater energy gap. Moreover, it is preferable to use the compound represented by the general formula (1) as a hole transporting material of a device using a triplet emissive material. This is because the compound represented by the general formula (1) has a high triplet level and therefore does not cause the problem with conventional hole transporting materials having a benzidine skeleton that leak of triplet excitation energy occurs and luminance efficiency drops if the materials are in contact directly with an emissive layer containing a triplet emitter dopant because of the low triplet levels of the materials.

Moreover, because of the presence of an amine skeleton bonded via a linking group L to the 3-position of a carbazole skeleton, the compound represented by the general formula (1) has a reduced ionization potential and exhibits better hole injection and transporting properties compared with the conventional compounds disclosed in Patent Document 3. A light emitting device with high efficiency and long lifetime can be obtained because the electron blocking property is also improved. When there is no linking group L and an amine skeleton is bonded to a carbazole skeleton directly, the electron blocking property deteriorates and the durable life decreases. Moreover, the triplet level also drops to cause decrease in luminance efficiency when being combined with an emissive layer containing a triplet emitter dopant. Furthermore, the amorphous property is improved and the stability of a thin film is increased by the formation of an asymmetric structure.

When being formed of a plurality of hole transporting layers, it is preferable that a hole transporting layer containing the compound represented by the general formula (1) is in contact with an emissive layer directly. This is because the compound represented by the general formula (1) has high electron blocking property and therefore can prevent the invasion of electrons flowing out of the emissive layer. Moreover, the compound represented by the general formula (1) has a high triplet level and therefore also has an effect of trapping the excitation energy of a triplet emissive material. Accordingly, it is preferable that a hole transporting layer containing the compound represented by the general formula (1) is in contact with an emissive layer also when a triplet emissive material is contained in the emissive layer.

The hole transporting layer may be formed of only the compound represented by formula (1) or alternatively may be incorporated with other materials as long as the effects of the present invention are not impaired. In this case, examples of the other materials to be used include benzidine derivative such as 4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB), and bis(N,N'-diphenyl-4-aminophenyl)-N,N-diphenyl-4,4'-diamino-1,1'-biphenyl (TPD232), materials called starburst arylamines such as 4,4',4"-tris(3-methylphenyl (phenyl)amino)triphenylamine (m-MTDATA) and 4,4',4"-tris(1-naphthyl (phenyl)amino)triphenylamine (1-TNATA), biscarbazole derivatives such as bis(N-arylcarbazole) or bis(N-alkylcarbazole), heterocyclic compounds, such as pyrazoline derivatives, stilbene-based compounds, hydrazone-based compounds, benzofuran derivatives, thiophene derivatives, oxadiazole derivatives, phthalocyanine derivatives, and porphyrin derivatives, and polymers such as polycarbonate having the above-provided monomers as side chains, styrene derivatives, polythiophene, polyaniline, polyfluorene, polyvinylcarbazole, and polysilane.

The emissive layers may be in the form of a single layer or a plurality of layers, each of which is formed of an emissive material (host material, dopant material), and this may be a mixture of the host material and the dopant material, or the host material alone. That is, in the light emitting device of the present invention, only the host material or the dopant material may emit light, or both of the host material and the dopant material emit light, in each emissive layer. From the viewpoints that electric energy is efficiently utilized, and light emission at high color purity is obtained, it is preferable that the emissive layer includes a mixture of the host material and the dopant material. In addition, the host material and the dopant material may be one kind or a combination of a plurality of kinds, respectively. The dopant material may be contained in a whole host material, or may be partially contained therein. The dopant material may be laminated, or may be dispersed. The dopant material can control an emitted color. Since when the amount of the dopant material is too large, concentration quenching occurs, it is used preferably in an amount of 20% by weight or less, further preferably 10% by weight or less relative to the host material. As a doping method, the dopant material can be co-evaporated with the host material, or the dopant material may be mixed with the host material in advance to be co-evaporated simultaneously.

Besides the compound represented by the general formula (1), examples of the emissive material that can be used include, but are not particularly limited to, fused ring derivatives such as anthracene and pyrene, metal chelated oxinoid compounds including tris(8-quinolinolate)aluminum, bisstyryl derivatives such as bisstyrylanthracene derivatives and distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, oxadiazole derivatives, thiadiazolopyridine derivatives, dibenzofuran derivatives, carbazole derivatives, and indolocarbazole derivatives and, as a polymer series, polyphenylenevinylene derivatives, polyparaphenylene derivatives, and polythiophene derivatives, which have hitherto been known as a light emitting body.

The host material contained in the emissive material need not be restricted to only one type of compound, and a plurality of compounds of the present invention may be used in admixture or a compound of the present invention may be used in an admixture with one or more other host materials. Examples of the host material which can be mixed include, but are not particularly limited to, compounds having a fused aryl ring such as naphthalene, anthracene, phenanthrene, pyrene, chrysene, naphthacene, triphenylene, perylene, fluoranthene, fluorene, and indene, and derivatives thereof, aromatic amine derivatives such as N,N'-dinaphthyl-N,N'-diphenyl-4,4'-diphenyl-1,1'-diamine, metal chelated oxinoid compounds including tris(8-quinolinato)aluminum (III), bisstyryl derivatives such as distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives, and triazine derivatives and, as a polymer series, polyphenylenevinylene derivatives, polyparaphenylene derivatives, polyfluorene derivatives, polyvinylcarbazole derivatives, and polythiophene derivatives. Especially, metal chelated oxinoid compounds, dibenzofuran derivatives, carbazole derivatives, indolocarbazole derivatives, triazine derivatives, etc. are suitably used as a host which is used when the emissive layer performs triplet emission (phosphorescence emission).

Preferably, the triplet emissive material to be used as a dopant is a metal complex compound containing at least one metal selected from the group consisting of iridium (Ir), ruthenium (Ru), palladium (Pd), platinum (Pt), osmium (Os), and rhenium (Re). It is preferable that the ligand has a nitrogen-containing aromatic heterocyclic ring such as a phenylpyridine skeleton or a phenylquinoline skeleton. However, the complex is not limited thereto, and a suitable complex is selected in context with an emitted color, a device performance and a host compound to be required. Specific examples thereof include a tris(2-phenylpyridyl) iridium complex, a tris{2-(2-thiophenyl)pyridyl} iridium complex, a tris{2-(2-benzothiophenyl)pyridyl} iridium complex, a tris(2-phenylbenzothiazole) iridium complex, a tris(2-phenylbenzooxazole) iridium complex, a trisbenzoquinoline iridium complex, a bis(2-phenyl pyridyl)(acetylacetonato) iridium complex, a bis{2-(2-thiophenyl)pyridyl} iridium complex, a bis{2-(2-benzothiophenyl)pyridyl}(acetylacetonato) iridium complex, a bis(2-phenylbenzothiazole)(acetylacetonato) iridium complex, a bis(2-phenylbenzooxazole)(acetylacetonato) iridium complex, a bisbenzoquinoline(acetylacetonato) iridium complex, a bis{2-(2,4-difluorophenyl)pyridyl}(acetylacetonato) iridium complex, a tetraethylporphyrin platinum complex, a {tris(thenoyltrifluoroacetone)-mono(1,10-phenanthroline)} europium complex, a {tris(thenoyltrifluoroacetone)-mono(4,7-diphenyl-1,10-phena nthroline)} europium complex, a {tris(1,3-diphenyl-1,3-propanedione)-mono(1,10-phenanthroli ne)} europium complex, and a trisacetylacetone terbium complex. Moreover, a phosphorescence dopant disclosed in JP-A-2009-130141 is also used suitably. Although not limited to these, an iridium complex or a platinum complex is used preferably because highly efficient light can be obtained easily.

Regarding the above-described triplet emissive materials to be used as a dopant material, only one material may be contained in an emissive layer or, alternatively, two or more materials may be used in admixture. When two or more triplet emissive materials are used, it is preferred that the total weight of the dopant materials is 20% by weight or less relative to the host material.

The emissive layer may further contain a third component for adjusting the carrier balance in the emissive layer or for stabilizing the layer structure of the emissive layer in addition to the above-described host material and the triplet emissive material. A material that does not cause interaction between a host material made of the above-described compound having a carbazole skeleton and a dopant material made of a triplet emissive material is chosen as the third component.

Preferable host and dopant in a triplet emission system are not particularly limited, and specific examples thereof include the following.

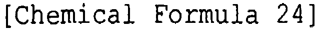

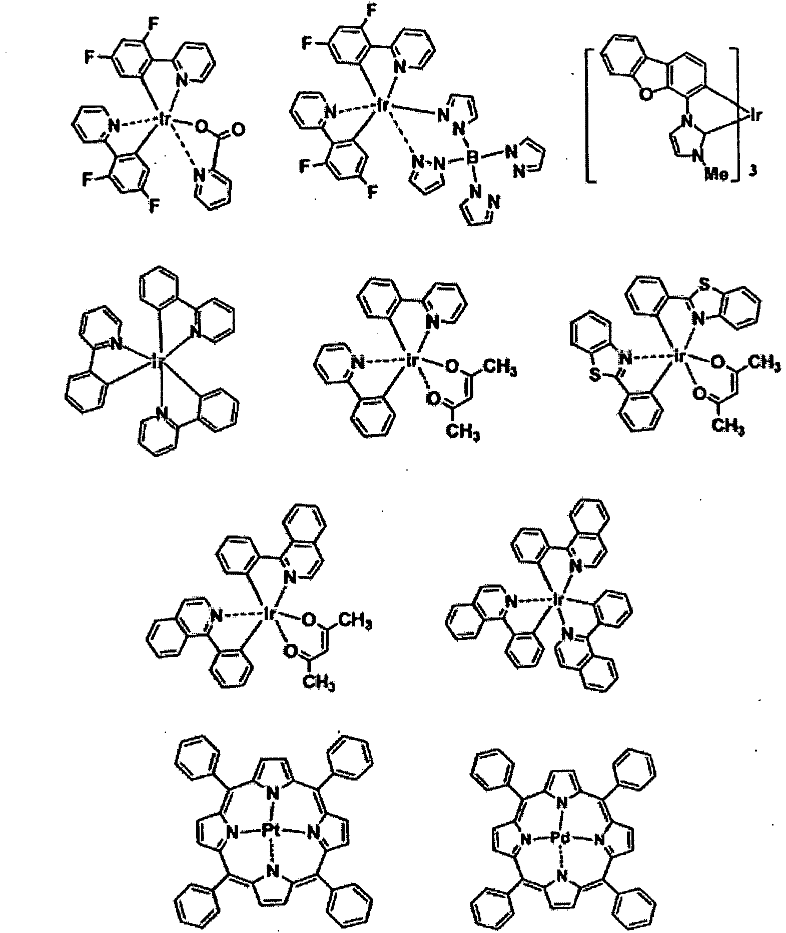

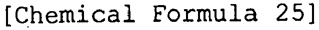

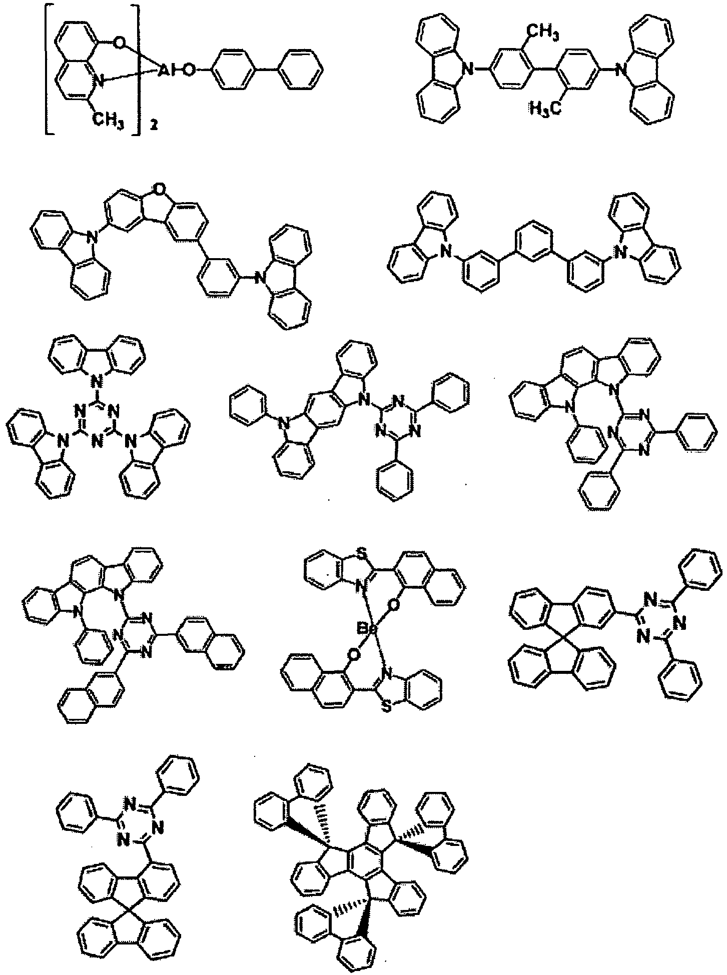

In the present invention, the electron transporting layer is a layer in which electrons are injected from the cathode and, further, which transports the electrons. It is desired that the electron transporting layer has a high electron injection efficiency, and efficiently transports injected electrons. For this reason, it is required that the electron transporting layer is constituted by a substance having great electron affinity and, moreover, great electron mobility and, further, excellent stability, and generating impurities that become a trap with difficulty at the time of manufacturing and at the time of use. Particularly, when layers are laminated at a large thickness, since a low-molecular compound is crystallized etc., and the film quality is easily deteriorated, a compound having a molecular weight of 400 or more which retains stable film quality is preferable. However, when transportation balance between holes and electrons is considered, if the electron transporting layer mainly plays a role of being able to inhibiting holes from the anode from flowing to the cathode side without recombination, even when the layer is constituted by a material having not so high electron transporting ability, the effect of improving luminance efficiency becomes equivalent to that when the layer is constituted by a material having high electron transporting ability. Therefore, the electron transporting layer in the present invention also includes a hole inhibition layer which can efficiently inhibit the transfer of holes as the same meaning.

Examples of the electron transporting material to be used for the electron transporting layer include fused polycyclic aromatic derivatives, such as naphthalene and anthracene, styryl-based aromatic derivatives typified by 4,4'-bis(diphenylethenyl)biphenyl, quinone derivatives, such as anthraquinone and diphenoquinone, phosphorus oxide derivatives, and various types of metal complexes, such as quinolinol complexes, e.g., tris(8-quinolinolate)aluminum(III), benzoquinolinol complexes, hydroxyazole complexes, azomethine complexes, tropolone metal complexes, and flavonol metal complexes. It is preferable to use a compound that includes an element selected from carbon, hydrogen, nitrogen, oxygen, silicon, and phosphorus and has a heteroaryl ring structure containing an electron-accepting nitrogen because it can reduce a driving voltage and a highly efficient light emission can be obtained.

The electron-accepting nitrogen referred to herein denotes a nitrogen atom which forms a multiple bond between adjoining atoms. Since nitrogen atoms have high electronegativity, the multiple bond has an electron-accepting nature. For this reason, an aromatic heterocyclic ring containing electron-accepting nitrogen has high electron affinity. An electron transporting material having electron-accepting nitrogen makes easier acceptance of electrons from a cathode having higher electron affinity, and lower voltage driving becomes possible. In addition, since supply of electrons to an emissive layer is increased and a recombining probability is increased, a luminance efficiency is increased.

Examples of the heteroaryl ring containing electron-accepting nitrogen include a pyridine ring, a pyrazine ring, a pyrimidine ring, a quinoline ring, a quinoxaline ring, a naphthylidine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, and a phenanthroimidazole ring.

Examples of preferred compounds having such a heteroaryl ring structure include benzimidazole derivatives, benzoxazole derivatives, benzthiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives such as bipyridine and terpyridine, quinoxaline derivatives and naphthylidine derivatives. Among these compounds, there can be preferably used imidazole derivatives such as tris(N-phenylbenzimidazol-2-yl)benzene; oxadiazole derivatives such as 1,3-bis[(4-tert-butylphenyl)1,3,4-oxadiazolyl]phenylene; triazole derivatives such as N-naphthyl-2,5-diphenyl-1,3,4-triazole; phenanthroline derivatives such as bathocuproine and 1,3-bis(1,10-phenanthrolin-9-yl)benzene; benzoquinoline derivatives such as 2,2'-bis(benzo[h]quinolin-2-yl)-9,9'-spirobifluorene; bipyridine derivatives such as 2,5-bis(6'-(2',2"-bipyridyl))-1,1-dimethyl-3,4-diphenylsilo le; terpyridine derivatives such as 1,3-bis(4'-(2,2':6'2"-terpyridinyl))benzene; and naphthylidine derivatives such as bis(1-naphthyl)-4-(1,8-naphthylidin-2-yl)phenylphosphine oxide in view of electron transporting ability. It is more preferable that such a derivative has a fused polycyclic aromatic skeleton because if so, then the glass transition temperature will increase and an effect of reducing the voltage of a light emitting device is great due to an increased electron mobility. Moreover, considering the improvement in durable life of a device, the easiness of synthesis, and easy availability of raw materials, it is particularly preferable that the fused polycyclic aromatic skeleton is an anthracene skeleton, a pyrene skeleton, or a phenanthroline skeleton. While the electron transporting material may be used alone, two or more kinds of the electron transporting materials may be used in combination, or one or more kinds of other electron transporting materials may be used in a combination with the electron transporting material.

Preferable electron transporting materials are not particularly limited, and specific examples thereof include the following.

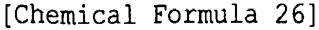

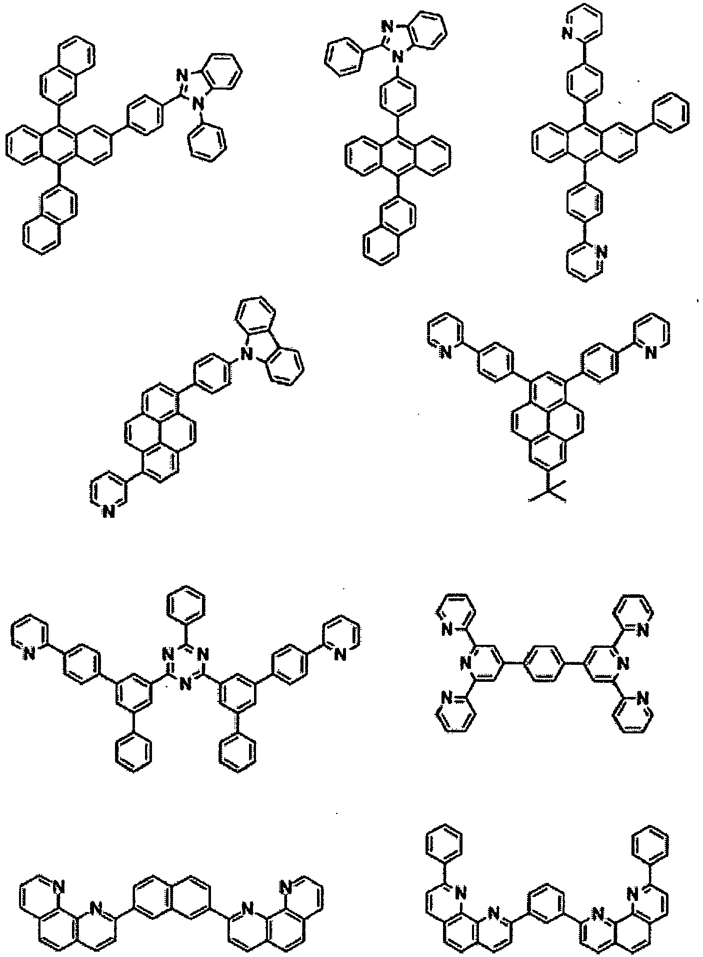

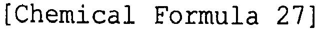

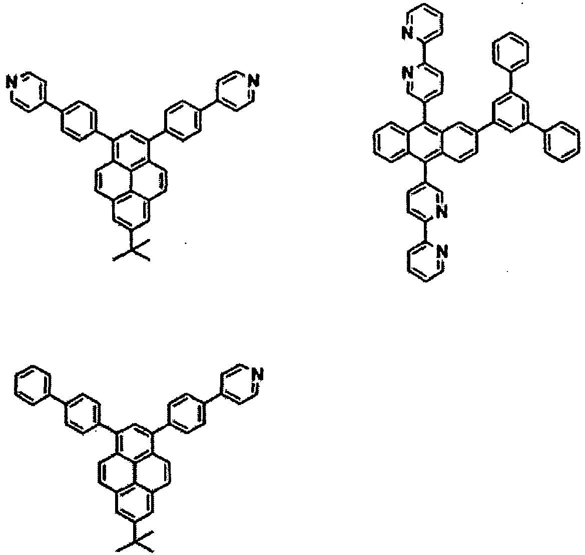

While the electron transporting material may be used alone, two or more kinds of the electron transporting materials may be used in combination, or one or more kinds of other electron transporting materials may be used in a combination with the electron transporting material. Moreover, a donor compound may be contained. The donor compound denotes a compound which makes easier electron injection into the electron transporting layer from the cathode or the electron injection layer and, further, improves the electric conductivity of the electron transporting layer, by improving an electron injection barrier.

Preferable examples of the donor compound include an alkali metal, an inorganic salt containing an alkali metal, a complex of an alkali metal and an organic substance, an alkaline earth metal, an inorganic salt containing an alkaline earth metal, or a complex of an alkaline earth metal and an organic substance. Examples of the preferable kind of the alkali metal and the alkaline earth metal include alkali metals such as lithium, sodium, potassium, rubidium, and cesium, and alkaline earth metals such as magnesium, calcium, cerium, and barium which have a low work function and have a great effect of improving electron transporting ability.

In addition, since evaporation in vacuum is easy, and handling is excellent, the donor compound is preferably in the state of an inorganic salt or a complex with an organic substance rather than a metal single substance. Moreover, from the viewpoints of improvement in easiness in handling in the atmospheric air, and easiness in control of the concentration to be added, the donor compound is more preferably in the state of a complex with an organic substance. Examples of the inorganic salt include oxides such as LiO and Li₂O, nitrides, fluorides such as LiF, NaF, and KF, and carbonates such as Li₂CO₃, Na₂CO₃, K₂CO₃, Rb₂CO₃, and Cs₂CO₃. Preferable examples of the alkali metal or alkaline earth metal include lithium and cesium from the viewpoint that a great low-voltage driving effect can be obtained. In addition, preferable examples of the organic substance in complexes with an organic substance include quinolinol, benzoquinolinol, pyridylphenol, flavonol, hydroxyimidazopyridine, hydroxybenzoazole, and hydroxytriazole. Especially, a complex of an alkali metal and an organic substance is preferred from the viewpoint that the effect of reducing the voltage of a light emitting device is greater, a complex of lithium and an organic substance is more preferred from the viewpoints of easiness in synthesis and thermal stability as well, and lithium quinolinol, which can be obtained relatively inexpensively, is particularly preferred.

The ionization potential of the electron transporting layer is not particularly limited, and is preferably 5.8 eV or more and 8.0 eV or less, and more preferably 6.0 eV or more and 7.5 eV or less.

Examples of a method of forming each of the aforementioned layers constituting the light emitting device include, but are not particularly limited to, resistance heating evaporation, electron beam evaporation, sputtering, a molecular lamination method, and a coating method, but usually, resistance heating evaporation or electron beam evaporation is preferable from the viewpoint of device property.

The thickness of the organic layer depends on the resistance value of an emissive substance and, therefore, it cannot be limited, but it is preferably 1 to 1000 nm. The film thickness of each of the emissive layer, the electron transporting layer and the hole transporting layer is preferably 1 nm or more and 200 nm or less, more preferably 5 nm or more and 100 nm or less.

The light emitting device of the present invention has a function of being able to convert electric energy into light. Herein, a direct current is mainly used as the electric energy, but a pulse current or an alternate current can also be used. A current value and a voltage value are not particularly limited, but when the power consumed and life of the device are considered, they should be selected so that the maximum luminance is obtained by energy as low as possible.

The light emitting device of the present invention is used suitably as a display that performs displays in a matrix and/or segment system.

In the matrix system, pixels for display are arranged two-dimensionally such as lattice-like arrangement or mosaic-like arrangement, and the collection of pixels displays letters and images. The shape and size of the pixel are determined depending on utility. For example, for displaying images and letters on personal computers, monitors and televisions, a square pixel being 300 µm or less at each side is usually used and, in the case of a large display such as a display panel, a pixel being millimeter order at each side is used. In the case of a monochromatic display, pixels having the same color may be arranged, and in the case of a color display, pixels having red, green and blue are arranged to perform display. In this case, typically, there are a delta type and a stripe type. A method of driving this matrix may be either a passive matrix driving method or an active matrix. The passive matrix driving has a simple structure, but when operation property is considered, the active matrix is more excellent in some cases, and it is necessary to use them properly depending on utility.

The segment system in the present invention is a system by which a pattern is formed so as to display predetermined information, and a region determined by arrangement of this pattern is made to emit light. Examples thereof include time and temperature displays in digital watches and thermometers, operating-state displays in audio equipment, microwave ovens and so on, and panel displays of automobiles. The above-mentioned matrix display and segment display may exist together in the same panel.

The light emitting device of the present invention can also be preferably used as backlight of various instruments. Backlight is used mainly for the purpose of improving the visibility of display apparatuses which do not emit light by themselves, and is used in liquid crystal display equipment, clocks, audio equipment, automobile panels, display panels, signs, etc. Particularly, the light emitting device of the present invention is preferably used in backlight for liquid crystal display apparatuses, inter alia, for personal computers which are studied to be thinned, and can provide backlight thinner and lighter than conventional products.

### [EXAMPLES]

The present invention will be described by way of Examples, but the present invention is not limited thereto. In addition, the number of a compound in each of Examples described below indicates the number of the aforementioned compound.

### Synthesis Example 1

### Synthesis of Compound [71] (HT-1)

A mixed solution of 20.9 g of 3, 6-dibromophenylcarbazole, 15.0 g of phenylcarbazole-3-boronic acid, 366 mg of bis(triphenylphosphine)palladium (II) dichloride, 105 ml of a 1M aqueous sodium carbonate solution, and 260 ml of dimethoxyethane was refluxed for 5 hours under a nitrogen flow. After cooling to room temperature, extraction with toluene was conducted. The organic layer was washed with water twice, dried over magnesium sulfate, and evaporated. The resultant concentrate was purified by silica gel column chromatography and then vacuum-dried to obtain 13.5 g of 6-bromo-9,9'H-3,3'-bicarbazole.

Next, a mixed solution of 5.3 g of 6-bromo-9,9'H-3,3'-bicarbazole, 3.0 g of 4-(diphenylamino)phenylboronic acid, 66 mg of bis(triphenylphosphine)palladium (II) dichloride, 21 ml of a 1M aqueous sodium carbonate solution, and 47 ml of dimethoxyethane was refluxed for 5 hours under a nitrogen flow. After cooling to room temperature, water was poured in and followed by filtration, and the solid collected by the filtration was vacuum-dried. The resultant solid was purified by silica gel column chromatography, vacuum-dried, and then extracted with toluene. The organic layer was washed with water twice, dried over magnesium sulfate, and evaporated. The resultant concentrate was purified by silica gel column chromatography, then recrystallized twice from a mixed solvent of toluene and methanol, and vacuum-dried. Thus, 1. 3 g of white powder was obtained.

¹H-NMR analytical results of the resulting powder are as follows and reveal that the resulting white crystal is Compound [71] (HT-1).
¹H-NMR(CDCl₃ (d=ppm)): 7.04-7.14 (m, 8H), 7.30-7.61 (m, 12H), 7.67-7.89 (m, 11H), 7.91 (d, 2H, 9.99), 8.39 (d, 1H, d = 7.83 Hz), 8.72 (m, 2H, d = 1.62), 8.82 (d, 1H, d = 1.35).

In addition, Compound [71] was used as a light emitting device material after sublimation purification was performed at about 350°C under a pressure of 1 x 10⁻³ Pa using an oil diffusion pump. The HPLC purity (area % at a measurement wavelength of 254 nm) was 99.8% before sublimation purification, and 99.9% after sublimation purification.

### Example 1

A glass substrate with an ITO transparent electrically conductive film deposited thereon in a thickness of 165 nm (manufactured by GEOMATEC Co., Ltd., 11Ω/□, sputtered product) was cut into 38 x 46 mm, and then subjected to etching. The resulting substrate was ultrasound-washed with "SEMICOCLEAN 56" (trade name, manufactured by Furuuchi Chemical Corporation) for 15 minutes, and then washed with ultrapure water. This substrate was treated with UV-ozone for 1 hour immediately before manufacturing of a device, and placed in a vacuum evaporation apparatus, and the air was evacuated until the degree of vacuum in the apparatus was 5 x 10⁻⁴ Pa or lower. By a resistance heating method, HI-1 was evaporated as a hole injection layer in a thickness of 10 nm. Then, HT-1 was evaporated as a hole transporting layer in a thickness of 60 nm. Then, Compound H-1 and Compound D-1 were used as a host material and as a dopant material, respectively, and were evaporated as an emissive layer in a thickness of 40 nm so that the doping concentration of the dopant material was 5% by weight. Then, Compound E-1 was laminated as an electron transporting layer in a thickness of 20 nm.

Then, lithium fluoride was evaporated in a thickness of 0.5 nm and aluminum was evaporated in a thickness of 60 nm to form a cathode, so that a 5 x 5 mm square device was manufactured. The film thickness referred to herein was an indicated value of a crystal oscillation film thickness monitor. When this light emitting device was direct-current driven at 10 mA/cm², blue light emission at a driving voltage of 4.8 V and an external quantum efficiency of 4.8% was obtained. This device was set to an initial luminance of 1000 cd/m² and the durable life thereof was measured. It was thereby found that the time required by a 20% reduction from the initial luminance was 450 hours. Compounds HI-1, HT-1, H-1, D-1, and E-1 are the compounds shown below.

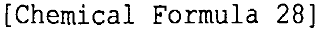

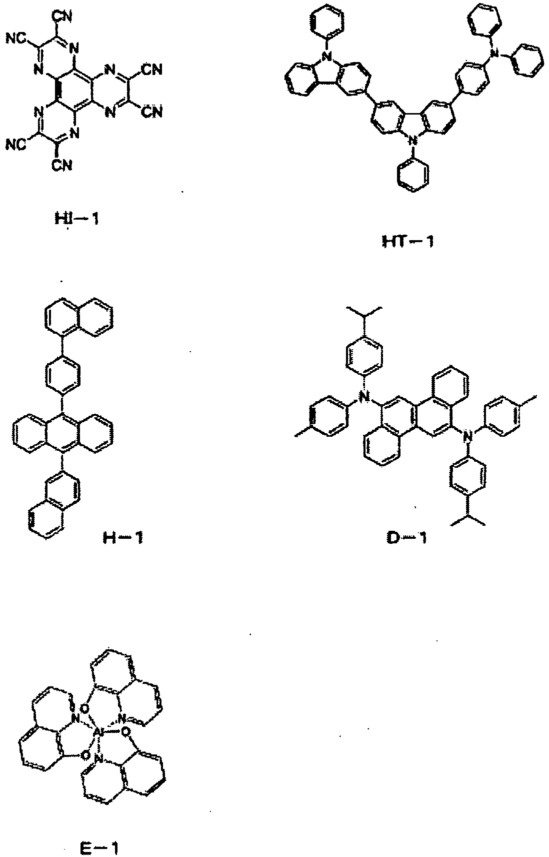

### Examples 2 to 19

According to the same manner as in Example 1 except that materials described in Table 1 were used as a hole transporting layer, light emitting devices were manufactured and evaluated. The results are shown in Table 1. HT-2 to HT-19 are compounds shown below.

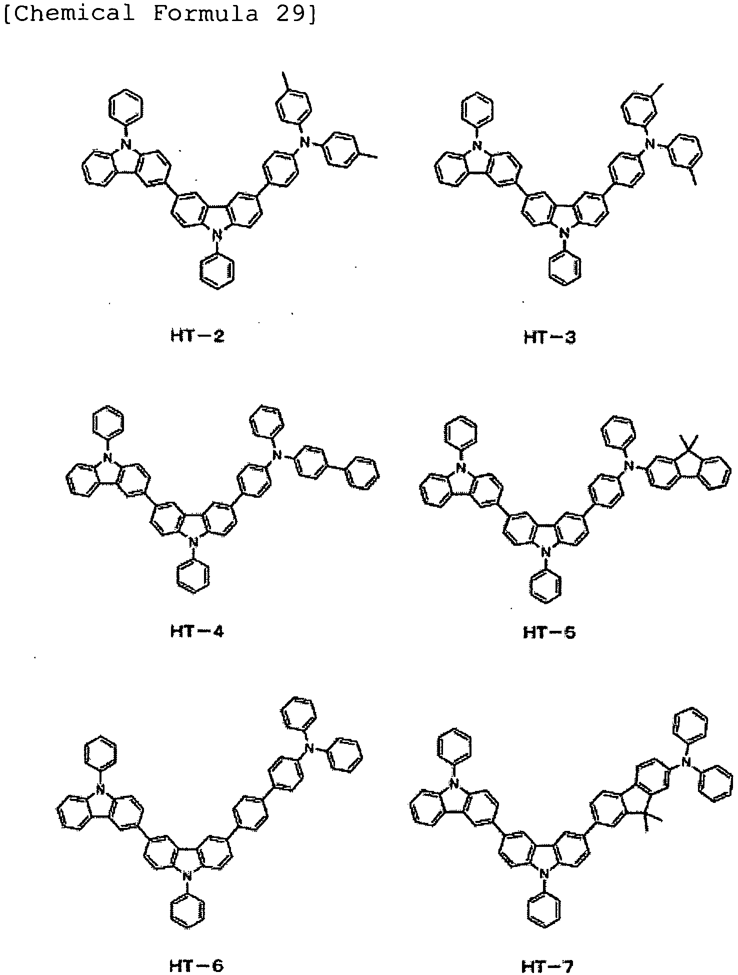

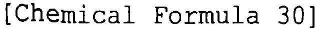

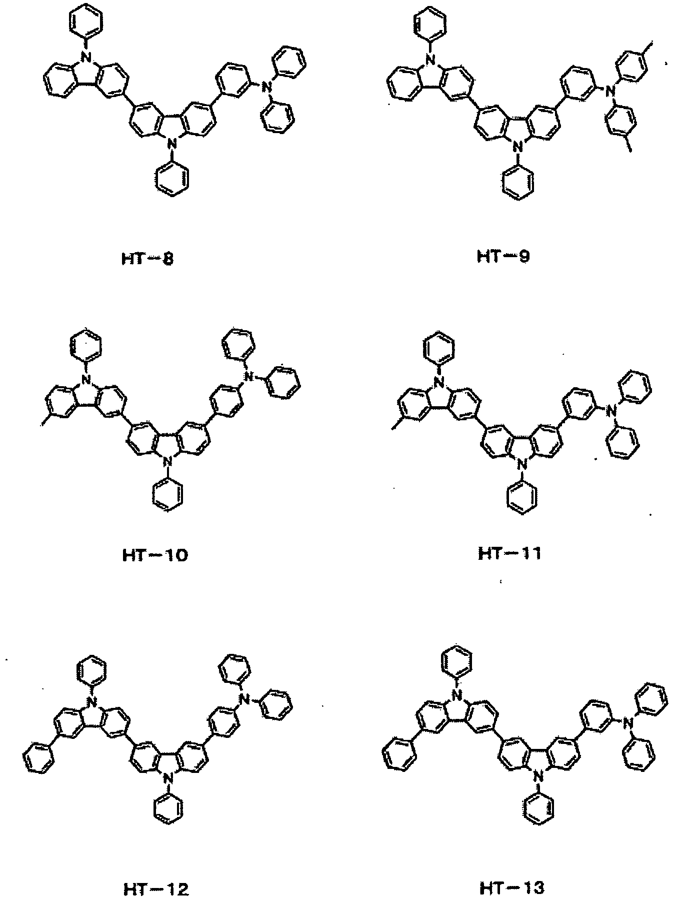

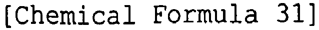

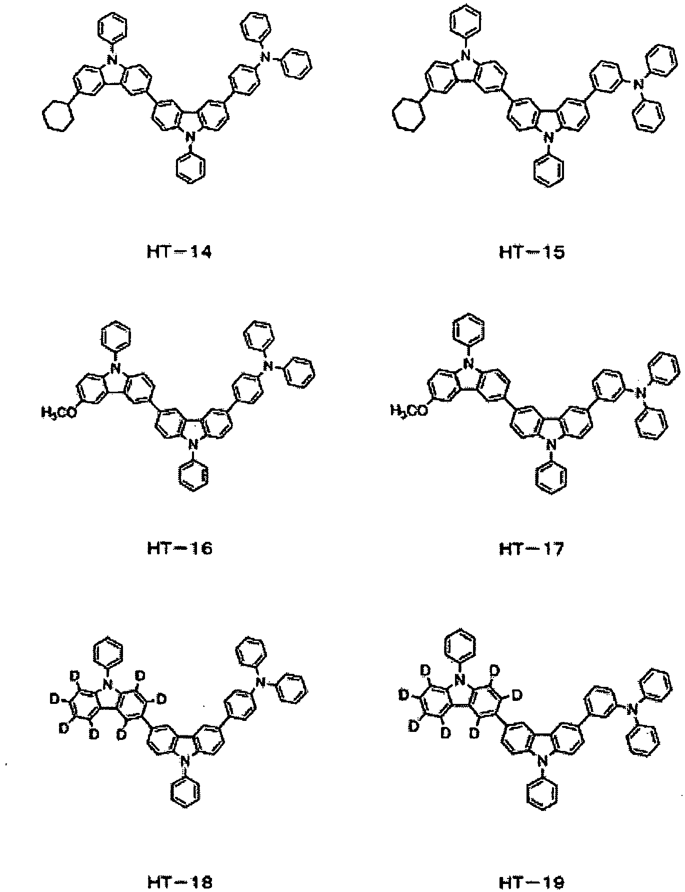

### Comparative Examples 1 to 7

According to the same manner as in Example 1 except that materials described in Table 1 were used as a hole transporting layer, light emitting devices were manufactured and evaluated. The results are shown in Table 1. HT-20 to HT-26 are compounds shown below.

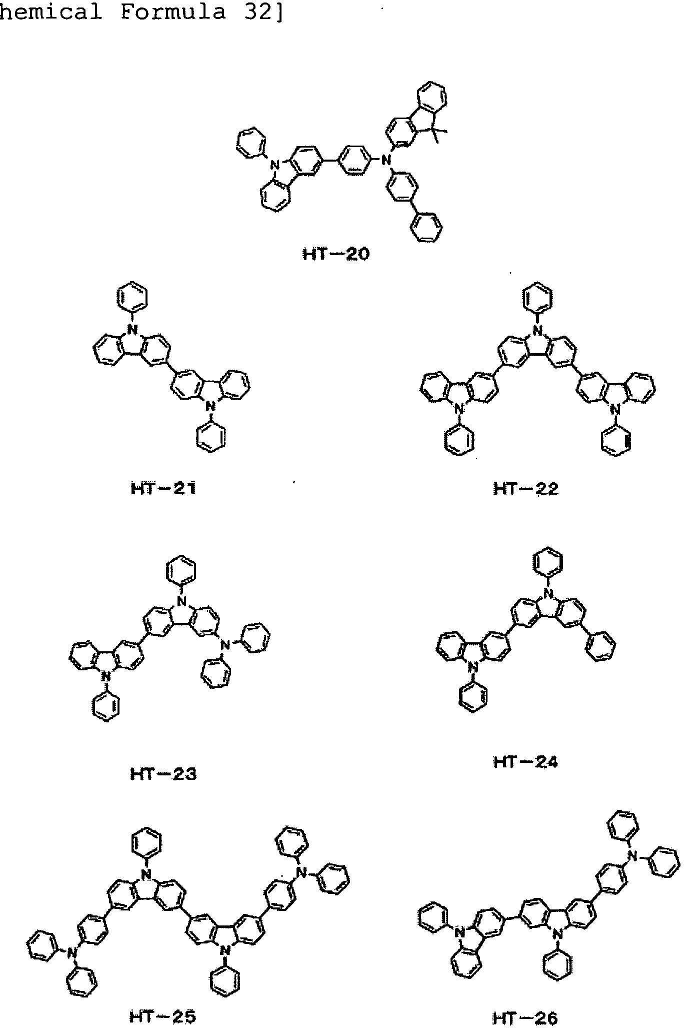

### Example 20

A glass substrate with an ITO transparent electrically conductive film deposited thereon in a thickness of 165 nm (manufactured by GEOMATEC Co., Ltd., 11Ω/□, sputtered product) was cut into 38 x 46 mm, and then subjected to etching. The resulting substrate was ultrasound-washed with "SEMICOCLEAN 56" (trade name, manufactured by Furuuchi Chemical Corporation) for 15 minutes, and then washed with ultrapure water. This substrate was treated with UV-ozone for 1 hour immediately before manufacturing of a device, and placed in a vacuum evaporation apparatus, and the air was evacuated until the degree of vacuum in the apparatus was 5 x 10⁻⁴ Pa or lower. By a resistance heating method, HI-1 was evaporated as a hole injection layer in a thickness of 10 nm. Then, HT-1 was evaporated as a hole transporting layer in a thickness of 60 nm. Then, Compound H-2 and Compound D-2 were used as a host material and as a dopant material, respectively, and were evaporated as an emissive layer in a thickness of 40 nm so that the doping concentration of the dopant material was 10% by weight. Then, Compound E-2 was laminated as an electron transporting layer in a thickness of 20 nm.

Then, lithium fluoride was evaporated in a thickness of 0.5 nm and aluminum was evaporated in a thickness of 60 nm to form a cathode, so that a 5 x 5 mm square device was manufactured. The film thickness referred to herein was an indicated value of a crystal oscillation film thickness monitor. When this light emitting device was direct-current driven at 10 mA/cm², green light emission at a driving voltage of 3. 7 V and an external quantum efficiency of 13.5% was obtained. This device was set to an initial luminance of 1000 cd/m² and the durable life thereof was measured. It was thereby found that the time required by a 20% reduction from the initial luminance was 250 hours. Compounds H-2, D-2, and E-2 are compounds shown below.

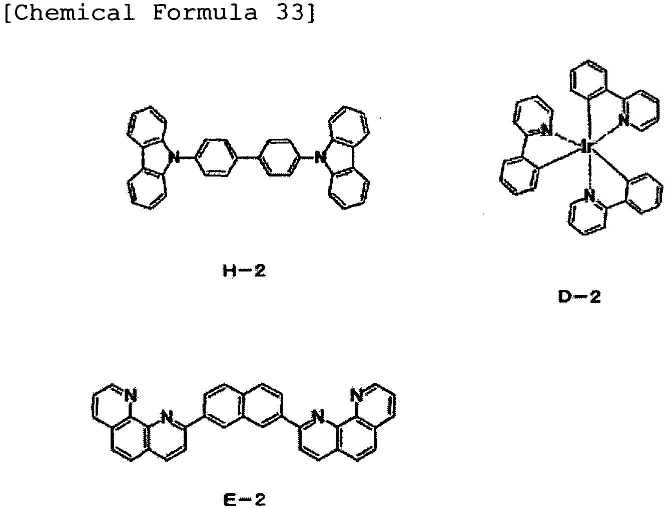

### Examples 21 to 38

According to the same manner as in Example 20 except that materials described in Table 2 were used as a hole transporting layer, light emitting devices were manufactured and evaluated.

The results are shown in Table 2.

### Comparative Examples 8 to 14

According to the same manner as in Example 20 except that compounds described in Table 2 were used as a hole transporting layer, light emitting devices were manufactured and evaluated. The results are shown in Table 2.

### Examples 39 to 57

According to the same manner as in Example 20 except that materials described in Table 3 were used as a hole transporting layer, a host material, and a dopant material, light emitting devices were manufactured and evaluated. The results are shown in Table 3. Compounds H-3 and D-3 are compounds shown below.

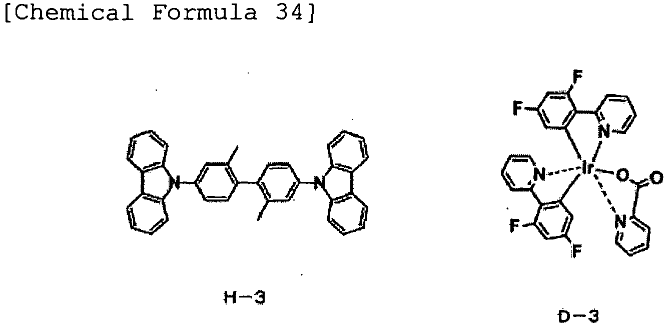

### Comparative Examples 15 to 21

According to the same manner as in Example 20 except that compounds described in Table 3 were used as a hole transporting layer, a host material, and a dopant material, light emitting devices were manufactured and evaluated. The results are shown in Table 3.

### Examples 58 to 76

According to the same manner as in Example 20 except that materials described in Table 4 were used as a hole transporting layer, a host material, and a dopant material, light emitting devices were manufactured and evaluated. The results were shown in Table 4. Compounds H-4 and D-4 are compounds shown below.

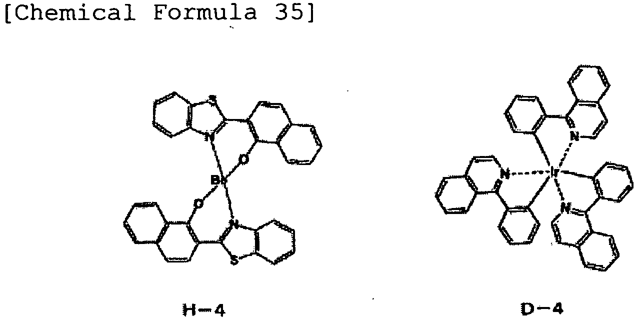

### Comparative Examples 22 to 28

According to the same manner as in Example 20 except that compounds described in Table 4 were used as a hole transporting layer, a host material, and a dopant material, light emitting devices were manufactured and evaluated. The results are shown in Table 3.

**[Table 1]**

| | Hole transporting layer | Host material | Dopant material | Emitted color | Driving voltage (V) | External quantum efficiency (%) | Luminance 20% reduction lifetime (hour) |
|---|---|---|---|---|---|---|---|
| Example 1 | HT-1 | H-1 | D-1 | Blue | 4.8 | 4.8 | 450 |
| Example 2 | HT-2 | H-1 | D-1 | Blue | 4.7 | 4.8 | 440 |
| Example 3 | HT-3 | H-1 | D-1 | Blue | 4.7 | 4.8 | 430 |
| Example 4 | HT-4 | H-1 | D-1 | Blue | 4.9 | 4.7 | 430 |
| Example 5 | HT-5 | H-1 | D-1 | Blue | 4.9 | 4.7 | 430 |
| Example 6 | HT-6 | H-1 | D-1 | Blue | 4.9 | 4.5 | 440 |
| Example 7 | HT-7 | H-1 | D-1 | Blue | 4.9 | 4.4 | 430 |
| Example 8 | HT-8 | H-1 | D-1 | Blue | 5.0 | 4.8 | 440 |
| Example 9 | HT-9 | H-1 | D-1 | Blue | 4.9 | 4.8 | 430 |
| Example 10 | HT-10 | H-1 | D-1 | Blue | 4.8 | 4.7 | 435 |
| Example 11 | HT-11 | H-1 | D-1 | Blue | 4.9 | 4.8 | 440 |
| Example 12 | HT-12 | H-1 | D-1 | Blue | 4.9 | 4.8 | 430 |
| Example 13 | HT-13 | H-1 | D-1 | Blue | 4.9 | 4.9 | 430 |
| Example 14 | HT-14 | H-1 | D-1 | Blue | 4.8 | 4.8 | 440 |
| Example 15 | HT-15 | H-1 | D-1 | Blue | 4.8 | 4.8 | 430 |
| Example 16 | HT-16 | H-1 | D-1 | Blue | 4.9 | 4.6 | 435 |
| Example 17 | HT-17 | H-1 | D-1 | Blue | 4.9 | 4.7 | 430 |
| Example 18 | HT-18 | H-1 | D-1 | Blue | 4.8 | 4.8 | 430 |
| Example 19 | HT-19 | H-1 | D-1 | Blue | 4.9 | 4.9 | 440 |
| Comparative Example 1 | HT-20 | H-1 | D-1 | Blue | 5.0 | 4.3 | 280 |
| Comparative Example 2 | HT-21 | H-1 | D-1 | Blue | 5.4 | 4.0 | 120 |
| Comparative Example 3 | HT-22 | H-1 | D-1 | Blue | 5.0 | 4.3 | 280 |
| Comparative Example 4 | HT-23 | H-1 | D-1 | Blue | 5.1 | 3.8 | 220 |
| Comparative Example 5 | HT-24 | H-1 | D-1 | Blue | 5.2 | 3.9 | 130 |
| Comparative Example 6 | HT-25 | H-1 | D-1 | Blue | 5.1 | 4.0 | 200 |
| Comparative Example 7 | HT-26 | H-1 | D-1 | Blue | 5.4 | 3.8 | 210 |

**[Table 2]**

| | Hole transporting layer | Host material | Dopant material | Emitted color | Driving voltage (V) | External quantum efficiency (%) | Luminance 20% reduction lifetime (hour) |
|---|---|---|---|---|---|---|---|
| Example 20 | HT-1 | H-2 | D-2 | Green | 3.7 | 13.5 | 250 |
| Example 21 | HT-2 | H-2 | D-2 | Green | 3.8 | 13.4 | 245 |
| Example 22 | HT-3 | H-2 | D-2 | Green | 3.9 | 13.6 | 251 |
| Example 23 | HT-4 | H-2 | D-2 | Green | 3.8 | 13.5 | 190 |
| Example 24 | HT-5 | H-2 | D-2 | Green | 3.9 | 13.4 | 180 |
| Example 25 | HT-6 | H-2 | D-2 | Green | 3.9 | 13.4 | 180 |
| Example 26 | HT-7 | H-2 | D-2 | Green | 3.9 | 13.4 | 175 |
| Example 27 | HT-8 | H-2 | D-2 | Green | 3.8 | 13.5 | 250 |
| Example 28 | HT-9 | H-2 | D-2 | Green | 3.8 | 13.4 | 245 |
| Example 29 | HT-10 | H-2 | D-2 | Green | 3.9 | 13.6 | 220 |
| Example 30 | HT-11 | H-2 | D-2 | Green | 3.9 | 13.5 | 200 |
| Example 31 | HT-12 | H-2 | D-2 | Green | 3.9 | 13.5 | 250 |
| Example 32 | HT-13 | H-2 | D-2 | Green | 3.7 | 13.6 | 250 |
| Example 33 | HT-14 | H-2 | D-2 | Green | 3.8 | 13.1 | 190 |
| Example 34 | HT-15 | H-2 | D-2 | Green | 3.9 | 13.2 | 180 |
| Example 35 | HT-16 | H-2 | D-2 | Green | 3.8 | 13.2 | 190 |
| Example 36 | HT-17 | H-2 | D-2 | Green | 3.9 | 13.0 | 180 |
| Example 37 | HT-18 | H-2 | D-2 | Green | 3.8 | 13.5 | 250 |
| Example 38 | HT-19 | H-2 | D-2 | Green | 3.9 | 13.6 | 240 |
| Comparative Example 8 | HT-20 | H-2 | D-2 | Green | 4.0 | 8.0 | 180 |
| Comparative Example 9 | HT-21 | H-2 | D-2 | Green | 4.6 | 12.0 | 140 |
| Comparative Example 10 | HT-22 | H-2 | D-2 | Green | 4.1 | 12.2 | 180 |
| Comparative Example 11 | HT-23 | H-2 | D-2 | Green | 4.5 | 8.1 | 140 |
| Comparative Example 12 | HT-24 | H-2 | D-2 | Green | 4.5 | 12.1 | 110 |
| Comparative Example 13 | HT-25 | H-2 | D-2 | Green | 4.2 | 10.2 | 110 |
| Comparative Example 14 | HT-26 | H-2 | D-2 | Green | 4.6 | 11.1 | 100 |

**[Table 3]**

| | Hole transporting layer | Host material | Dopant material | Emitted color | Driving voltage (V) | External quantum efficiency (%) | Luminance 20% reduction lifetime (hour) |
|---|---|---|---|---|---|---|---|
| Example 39 | HT-1 | H-3 | D-3 | Blue | 4.1 | 13.0 | 200 |
| Example 40 | HT-2 | H-3 | D-3 | Blue | 4.2 | 13.0 | 205 |
| Example 41 | HT-3 | H-3 | D-3 | Blue | 4.4 | 12.9 | 195 |
| Example 42 | HT-4 | H-3 | D-3 | Blue | 4.4 | 12.4 | 150 |
| Example 43 | HT-5 | H-3 | D-3 | Blue | 4.6 | 12.3 | 148 |
| Example 44 | HT-6 | H-3 | D-3 | Blue | 4.5 | 12.3 | 150 |
| Example 45 | HT-7 | H-3 | D-3 | Blue | 4.6 | 12.2 | 145 |
| Example 46 | HT-8 | H-3 | D-3 | Blue | 4.1 | 13.0 | 200 |
| Example 47 | HT-9 | H-3 | D-3 | Blue | 4.2 | 13.0 | 205 |
| Example 48 | HT-10 | H-3 | D-3 | Blue | 4.4 | 12.9 | 195 |
| Example 49 | HT-11 | H-3 | D-3 | Blue | 4.4 | 12.9 | 185 |
| Example 50 | HT-12 | H-3 | D-3 | Blue | 4.6 | 12.9 | 190 |
| Example 51 | HT-13 | H-3 | D-3 | Blue | 4.1 | 12.8 | 195 |
| Example 52 | HT-14 | H-3 | D-3 | Blue | 4.2 | 12.5 | 150 |
| Example 53 | HT-15 | H-3 | D-3 | Blue | 4.4 | 12.4 | 145 |
| Example 54 | HT-16 | H-3 | D-3 | Blue | 4.4 | 12.2 | 150 |
| Example 55 | HT-17 | H-3 | D-3 | Blue | 4.6 | 12.3 | 148 |
| Example 56 | HT-18 | H-3 | D-3 | Blue | 4.5 | 12.3 | 200 |
| Example 57 | HT-19 | H-3 | D-3 | Blue | 4.6 | 12.1 | 200 |
| Comparative Example 15 | HT-20 | H-3 | D-3 | Blue | 5.0 | 8.0 | 120 |
| Comparative Example 16 | HT-21 | H-3 | D-3 | Blue | 5.6 | 11.5 | 50 |
| Comparative Example 17 | HT-22 | H-3 | D-3 | Blue | 5.1 | 11.4 | 125 |
| Comparative Example 18 | HT-23 | H-3 | D-3 | Blue | 5.7 | 6.0 | 70 |
| Comparative Example 19 | HT-24 | H-3 | D-3 | Blue | 5.8 | 11.7 | 60 |
| Comparative Example 20 | HT-25 | H-3 | D-3 | Blue | 5.7 | 10.2 | 70 |
| Comparative Example 21 | HT-26 | H-3 | D-3 | Blue | 5.9 | 10.8 | 80 |

**[Table 4]**

| | Hole transporting layer | Host material | Dopant material | Emitted color | Driving voltage (V) | External quantum efficiency (%) | Luminance 20% reduction lifetime (hour) |
|---|---|---|---|---|---|---|---|
| Example 58 | HT-1 | H-4 | D-4 | Red | 3.7 | 15.0 | 350 |
| Example 59 | HT-2 | H-4 | D-4 | Red | 3.8 | 15.1 | 340 |
| Example 60 | HT-3 | H-4 | D-4 | Red | 3.9 | 14.9 | 330 |
| Example 61 | HT-4 | H-4 | D-4 | Red | 3.8 | 14.8 | 300 |
| Example 62 | HT-5 | H-4 | D-4 | Red | 3.9 | 14.5 | 290 |
| Example 63 | HT-6 | H-4 | D-4 | Red | 3.9 | 14.9 | 280 |
| Example 64 | HT-7 | H-4 | D-4 | Red | 3.9 | 14.7 | 270 |
| Example 65 | HT-8 | H-4 | D-4 | Red | 3.8 | 14.4 | 280 |
| Example 66 | HT-9 | H-4 | D-4 | Red | 3.8 | 14.6 | 350 |
| Example 67 | HT-10 | H-4 | D-4 | Red | 3.9 | 14.2 | 290 |
| Example 68 | HT-11 | H-4 | D-4 | Red | 3.9 | 14.2 | 280 |
| Example 69 | HT-12 | H-4 | D-4 | Red | 3.9 | 14.3 | 280 |
| Example 70 | HT-13 | H-4 | D-4 | Red | 3.7 | 14.5 | 280 |
| Example 71 | HT-14 | H-4 | D-4 | Red | 3.8 | 14.3 | 250 |
| Example 72 | HT-15 | H-4 | D-4 | Red | 3.9 | 14.7 | 250 |
| Example 73 | HT-16 | H-4 | D-4 | Red | 3.8 | 14.5 | 280 |
| Example 74 | HT-17 | H-4 | D-4 | Red | 3.9 | 14.7 | 270 |
| Example 75 | HT-18 | H-4 | D-4 | Red | 3.8 | 14.6 | 350 |
| Example 76 | HT-19 | H-4 | D-4 | Red | 3.9 | 14.7 | 350 |
| Comparative Example 22 | HT-20 | H-4 | D-4 | Red | 4.3 | 11.7 | 240 |
| Comparative Example 23 | HT-21 | H-4 | D-4 | Red | 4.5 | 11.8 | 150 |
| Comparative Example 24 | HT-22 | H-4 | D-4 | Red | 4.2 | 11.9 | 250 |
| Comparative Example 25 | HT-23 | H-4 | D-4 | Red | 4.3 | 11.6 | 140 |
| Comparative Example 26 | HT-24 | H-4 | D-4 | Red | 4.4 | 11.4 | 120 |
| Comparative Example 27 | HT-25 | H-4 | D-4 | Red | 4.5 | 11.6 | 140 |
| Comparative Example 28 | HT-26 | H-4 | D-4 | Red | 4.5 | 11.5 | 160 |

## Claims

1. A light emitting device material comprising a compound represented by general formula (1): 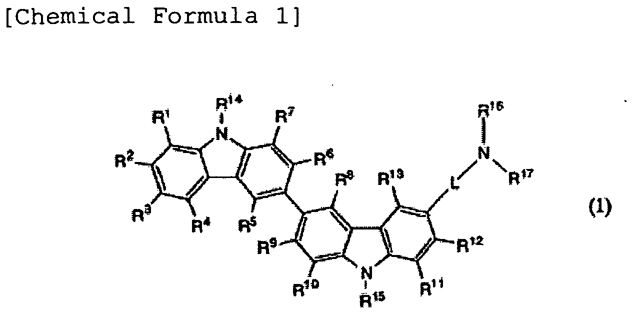 wherein R¹ to R¹³ may be the same or different and are each selected from the group consisting of hydrogen, deuterium, an alkyl group, a cycloalkyl group, an amino group, an aryl group, a heterocyclic group, a heteroaryl group, an alkenyl group, a cycloalkenyl group, an alkynyl group, an alkoxy group, an alkylthio group, an aryl ether group, an aryl thioether group, halogen, a cyano group, a carbonyl group, a carboxyl group, an oxycarbonyl group, a carbamoyl group, -P(=O)R¹⁸R¹⁹, and a silyl group; R¹⁸ and R¹⁹ are each an aryl group or a heteroaryl group; these substituents each may be further substituted and adjoining substituents may further form a ring; R¹⁴ and R¹⁵ may be the same or different and are each selected from the group consisting of an alkyl group, an aryl group, an alkenyl group, an alkylthio group, an arylthio group, a heterocyclic group, and a heteroaryl group; L is an alkylene group, an arylene group, or a heteroarylene group; R¹⁶ and R¹⁷ may be the same or different and are each selected from the group consisting of an alkyl group, an aryl group, an alkenyl group, an alkylthio group, an arylthio group, a heterocyclic group, and a heteroaryl group; and R¹⁴ to R¹⁷ each may be further substituted.

2. The light emitting device material according to claim 1, wherein the compound represented by the general formula (1) is an asymmetric carbazole dimer.

3. The light emitting device material according to claim 1 or 2, wherein in the general formula (1), R³ is hydrogen, deuterium, an alkyl group, a cycloalkyl group, an alkoxy group, an unsubstituted aryl group, or an unsubstituted heteroaryl group.

4. The light emitting device material according to any one of claims 1 to 3, wherein in the general formula (1), L is a phenylene group, a biphenylene group, a fluorenylene group, a furanylene group, a thienylene group, a dibenzofuranylene group, or a dibenzothiophenylene group.

5. The light emitting device material according to any one of claims 1 to 4, wherein in the general formula (1), R¹⁶ and R¹⁷ are each selected from the group consisting of a phenyl group, a biphenyl group, a fluorenyl group, a dibenzofuranyl group, and a dibenzothiophenyl group.

6. The light emitting device material according to any one of claims 1 to 5, wherein in the general formula (1), R¹⁴ and R¹⁵ are each selected from an aryl group or a heteroaryl group.

7. A light emitting device, in which an organic layer exists between an anode and a cathode and which emits light by means of electric energy, wherein the light emitting device contains the light emitting device material according to any one of claims 1 to 6 in the organic layer.

8. The light emitting device according to claim 7, wherein at least a hole transporting layer exists as the organic layer and the light emitting device contains a compound represented by general formula (1) in the hole transporting layer.

9. The light emitting device according to claim 7 or 8, wherein at least an emissive layer containing a triplet emissive material exists as the organic layer.
